# EUROPEAN PATENT APPLICATION

(11) **EP 4 786 586 A1**
(43) Date of publication of application: **05.08.2026**
(21) Application number: 23953774.9
(22) Date of filing: 28.09.2023
(51) Int. Cl.: C12N 15/09, C12N 15/63, C12N 15/113

(54) **NUCLEIC ACID MOLECULE THAT REGULATES GENE EXPRESSION USING RNA SPLICING MODULATOR**

(71) Applicant: Peking University, Beijing 100871 (CN); Beijing Biotune Medical Technology Co., Ltd., Beijing 100000 (CN)
(72) Inventor: GUO, Yuxuan, Beijing 100871 (CN); CHEN, Zhan, Beijing 100871 (CN); YANG, Luzi, Beijing 100871 (CN); YANG, Ke, Beijing 100000 (CN)
(74) Representative: Chung, Hoi Kan
(86) International application number: PCT/CN2023/122923
(87) International publication number: WO 2025/065630

(57) **Abstract**

A nucleic acid molecule that regulates gene expression using an RNA splicing modulator, a nucleic acid construct containing an alternative splicing regulatory element and a target gene located at the 3' end of the alternative splicing regulatory element, a transcript of the nucleic acid construct under different conditions, and a vector, recombinant virus, cell and pharmaceutical composition containing the nucleic acid construct or the transcript, a method for regulating the expression level of a target gene by using same, and the use thereof in gene therapy.

## Description

### TECHNICAL FIELD

The present disclosure relates generally to the fields of molecular biology and medicine, and more particularly, it concerns nucleic acid molecules for modulating gene expression using RNA splicing modulator.

### BACKGROUND

Many human diseases are related to genetic deficiencies. A promising approach to treating these diseases is gene therapy. In gene therapy, people deliver exogenous genetic material into target cells to intervene in the physiological functions of cells at the nucleic acid level, thereby ultimately treating the disease. For example, gene therapy can achieve the purpose of treating diseases by introducing normal genes to replace missing or abnormally mutated disease-causing genes, or by inhibiting the function of harmful endogenous genes through gene silencing technology. The research focus of gene therapy in the past 20 years has been mainly on developing and optimizing gene delivery systems. For example, engineering the adeno-associated virus (AAV) capsid can optimize its gene delivery efficiency and specific organs targeting. However, there is little research on the therapeutic genes delivered in gene therapy. These studies mainly focus on promoter technology, gene expression regulation technology, and 3' modulatory elements that limit expression to specific cell types.

The gene expression level is an important factor affecting the effect of gene therapy. If the expression level of the therapeutic gene is insufficient or not expressed, gene therapy cannot achieve the expected effect; on the other hand, if the expression level of the therapeutic gene is too high, that is, overexpression, gene therapy is likely to cause unexpected toxic side effects to the patient. How to regulate the expression level of genes after they are delivered into the body is a difficult and painful issue in gene therapy research. At present, relevant gene expression regulation technologies are extremely limited. One of the ideas is to regulate gene expression through drug-induced RNA splicing changes.

Currently, LMI070 remains the only RNA splicing modulatory drug known to be used for gene expression regulation, with no other alternatives available. On the other hand, the modulatory capacity of currently used RNA splicing modulatory drugs is also limited and needs to be further improved. In addition, the available methods for detecting changes in RNA splicing are limited, and more effective detection technologies need to be developed. Furthermore, the nucleic acid sequences in the prior art are relatively lengthy, and the intron sequences harboring key functions are undefined. It is necessary to develop more compact nucleic acid sequences and clarify the sequences that play a key role in the sequences through experiments. Another shortcoming is that the alternative splicing modulatory elements reported in existing literature are derived from cells cultured in vitro rather than organs in vivo, and lack adaptation and optimization to the physiological and pathological environment in vivo.

### SUMMARY

To address the above problems existing in the prior art, a technical solution disclosed in the present disclosure is provided.

In a first aspect of the present disclosure, an alternative splicing modulatory element is provided. The alternative splicing modulatory element comprises a first exon, a first intron, a pseudoexon, a second intron and a second exon sequentially from 5' end to 3' end; the pseudoexon comprises a nucleotide sequence having at least 95% identity to the sequence as shown in SEQ ID NO: 1; the junction between the pseudoexon and the second intron comprises a binding site for a small-molecule alternative splicing modifier drug; and the alternative splicing modulatory element comprises a sole start codon exclusively in the pseudoexon. The start codon may be selected from ATG, GTG, ACG, CTG, ATA or TTG, preferably ATG.

In one embodiment, the binding site for a small-molecule alternative splicing modifier drug comprises the nucleotide sequence ATGAGTA, wherein ATGA belongs to the pseudoexon and GTA belongs to the second intron; the binding site for a small-molecule alternative splicing modifier drug comprises the nucleotide sequence AGAGTA, wherein AGA belongs to the pseudoexon and GTA belongs to the second intron. The sequence of the first exon immediately followed by the sequence of the first intron is CAG, and the sequence of the first intron immediately following the sequence of the first exon is GTA; and/or, the sequence of the first intron immediately followed by the sequence of the pseudoexon is TAG, and the sequence of the pseudoexon immediately following the sequence of the first intron is GTT; and/or, the sequence of the second intron immediately followed by the sequence of the second exon is CAG, and the sequence of the second exon immediately following the sequence of the second intron is TTT.

In one embodiment, the first intron comprises a nucleotide sequence that has at least 95% (preferably 96%, 97%, 98%, 99% or 99.5%) identity to the sequences as shown in SEQ ID NOs: 19 and 20; the second intron comprises a nucleotide sequence that has at least 95% identity to the sequence as shown in SEQ ID NO: 21.

In one embodiment, the nucleotide length of the first intron is 41 to 500 bp, preferably 41 to 410 bp, preferably 41 to 140 bp, preferably 41 to 110 bp; the nucleotide length of the second intron is 60 to 569 bp, preferably 60 to 479 bp, preferably 60 to 209 bp, preferably 60 to 129 bp.

In one embodiment, the first intron comprises a nucleotide sequence that has at least 95% (preferably 96%, 97%, 98%, 99% or 99.5%) identity to the sequence as shown in any one of SEQ ID NOs: 3 to 5, 22 to 25; the second intron comprises a nucleotide sequence that has at least 95% (preferably 96%, 97%, 98%, 99% or 99.5%) identity to the sequence as shown in any one of SEQ ID NOs: 6 to 8, 26 to 32.

Preferred combinations include:
(a) the first intron comprises a nucleotide sequence having at least 95% (preferably 96%, 97%, 98%, 99% or 99.5%) identity to the sequence as shown in SEQ ID NO:5; the second intron comprises a nucleotide sequence having at least 95% (preferably 96%, 97%, 98%, 99% or 99.5%) identity to the sequence as shown in SEQ ID NO:8; or,
(b) the first intron comprises a nucleotide sequence having at least 95% (preferably 96%, 97%, 98%, 99% or 99.5%) identity to the sequence as shown in SEQ ID NO:4; the second intron comprises a nucleotide sequence having at least 95% (preferably 96%, 97%, 98%, 99% or 99.5%) identity to the sequence as shown in SEQ ID NO:7; or,
(c) the first intron comprises a nucleotide sequence having at least 95% (preferably 96%, 97%, 98%, 99% or 99.5%) identity to the sequence as shown in SEQ ID NO:3; and the second intron comprises a nucleotide sequence having at least 95% (preferably 96%, 97%, 98%, 99% or 99.5%) identity to the sequence as shown in SEQ ID NO:6.

In one embodiment, the first exon comprises a nucleotide sequence that has at least 95% (preferably 96%, 97%, 98%, 99% or 99.5%) identity to the sequence as shown in SEQ ID NO:2; and/or the second exon comprises a nucleotide sequence that has at least 95% (preferably 96%, 97%, 98%, 99% or 99.5%) identity to the sequence as shown in SEQ ID NO:9.

After combining the above-mentioned first exon and second exon nucleotide sequences, the alternative splicing modulatory element further preferably comprises a nucleotide sequence having at least 95% (preferably 96%, 97%, 98%, 99% or 99.5%) identity to the sequence as shown in any one of SEQ ID NOs: 10 to 12.

In the second aspect of the present disclosure, the present disclosure provides a nucleic acid molecule, which comprises the alternative splicing modulatory element as described in the first aspect of the present disclosure and a target gene positioned at the 3' end of the alternative splicing modulatory element of the present disclosure; wherein the target gene is one or more protein-encoding genes and/or non-coding RNA encoding genes , and the first start codon ATG of the protein-encoding gene is deleted.

In one embodiment, there is no nucleic acid sequence between the 3' end of the alternative splicing modulatory element and the 5' end of the target gene.

In one embodiment, there is a sequence encoding a protease cleavage site between the 3' end of the alternative splicing modulatory element and the 5' end of the target gene, wherein the protease cleavage site is cleaved by a mammalian protease or is self-cleaved, such as a 2A peptide, including but not limited to P2A (porcine teschovirus-1), T2A (thosea asigna virus), F2A (foot-and-mouth disease virus), E2A (equine rhinitis A virus), preferably P2A (SEQ ID NO: 13). Those skilled in the art will understand that in this embodiment, the number of bases of the sequence encoding the protease cleavage site between the 3' end of the alternative splicing modulatory element and the 5' end of the target gene should be 3n, where n is an integer greater than or equal to 1, to keep the reading frame of the target gene from being altered or shifted.

In one embodiment, the 5' end of the alternative splicing modulatory element is operably linked to a promoter sequence.

In one embodiment, the promoter sequence is a mammalian cell constitutive promoter, a mammalian cell specific promoter, a mammalian non-coding RNA promoter or a prokaryotic cell promoter.

In one embodiment, the promoter sequence is a mammalian cell constitutive promoter. Preferably, the promoter is CMV (cytomegalovirus), CAG (composed of cytomegalovirus enhancer and chicken β-actin promoter), CBG, EF1a (elongation factor-1α), PGK1 (phosphoglycerate kinase 1) or Ubc (ubiquitin C).

In one embodiment, the promoter sequence is a mammalian cell-specific promoter. Preferably, the promoter is Tnnt2 (cardiomyocytes), Nppa (atrial cardiomyocytes), Myl2 (ventricular cardiomyocytes), Mck (skeletal muscle cells), Nkx2.5 (cardiac progenitor cells), Syn (neurons), Mecp2 (neurons), TBG (hepatocytes), Pdx1 (pancreatic cells), K14 (skin keratinocytes), Rpe65 (retinal cells) or SP-C (lung epithelial cells).

In one embodiment, the promoter sequence is a mammalian non-coding RNA promoter. Preferably, the promoter is U6 or H1 (a eukaryotic RNA polymerase III-dependent promoter).

In one embodiment, the promoter sequence is a prokaryotic promoter. Preferably, the promoter is T7 (T7 phage), T3 (T3 phage), SP6 (SP6 phage).

In one embodiment, the nucleic acid molecules of the present disclosure further comprise a post-transcriptional modulatory element and/or a target sequence for a microRNA. Preferably, the post-transcriptional modulatory element (PRE) comprises a post-transcriptional modulatory element derived from hepatitis B (HPRE), bat (BPRE), ground squirrel (GSPRE), Arctic squirrel (ASPRE), duck (DPRE), chimpanzee (CPRE), woolly monkey (WMPRE) or woodchuck (WPRE). The target sequence of the microRNA is selected from the target sequence of miR122, miR199, miR7, miR148, miR1 or miR208. Optionally, the post-transcriptional modulatory element and/or the target sequence of the microRNA is disposed at the 3' end of the target gene.

In one embodiment, the nucleic acid molecule of the present disclosure further comprises a polyadenylation signal (polyA), optionally wherein the polyA is disposed at the 3' end of the target gene. Preferably, the polyA signal is SV40 polyA, human growth hormone (HGH) polyA, bovine growth hormone (BGH) polyA, β-globin polyA, α-globin polyA, ovalbumin polyA, κ-light chain polyA or synthetic polyA.

In one embodiment, the alternative splicing modulatory element is capable of being combined with a small-molecule alternative splicing modifier drug. Preferably, the small-molecule alternative splicing modifier drug is LMI070, a derivative having the same binding site as LMI070 on the alternative splicing modulatory element, or a pharmaceutically acceptable salt of LMI070; or, the small-molecule alternative splicing modifier drug is Risdiplam, a derivative having the same binding site as Risdiplam on the alternative splicing modulatory element, or a pharmaceutically acceptable salt of Risdiplam.

In one embodiment, the protein encoding gene is a gene encoding a nuclease, and/or a gene encoding a therapeutic protein, and/or a gene encoding a reporter or marker protein, and/or a gene encoding a non-coding RNA. Preferably, the nuclease includes a transcription activator-like effector nuclease (TALEN), a zinc finger nuclease (ZFN) or a CRISPR associated protein (Cas protein) or its functionally equivalent derivatives, and further preferably, the Cas protein includes a Cas9 protein, a Cas12 protein or a Cas13 protein. Preferably, the therapeutic protein includes YAP (Yes-associated protein), LMNA (lamin), RPE65 (all-trans retinol ester isomerase), SMN1 (survival motor neuron gene 1 protein), FVIII (coagulation factor VIII), FIX (coagulation factor IX) or their functionally equivalent derivatives. Preferably, the marker protein includes eGFP (enhanced green fluorescent protein), tdTomato (red fluorescent protein variant), mCherry (cherry red fluorescent protein), Luciferase (luciferase), SEAP (secreted placental alkaline phosphatase), CAT (catalase), GST (glutathione sulfhydryltransferase), β-GUS (β-glucuronidase), β-Gal (β-galactosidase) or their functionally equivalent derivatives. Preferably, the non-coding RNA includes at least one of miRNA, shRNA or LncRNA having gene inhibition function.

In one embodiment, the method for constructing the nucleic acid molecule disclosed herein comprises connecting the target gene with the 5'-end start codon removed to the 3' end of the alternative splicing modulatory element disclosed herein, so that the target gene and the start codon in the alternative splicing modulatory element use the same reading frame.

In the third aspect of the present disclosure, a transcript of the nucleic acid molecule is provided according to the second aspect of the present disclosure in the presence of a small-molecule alternative splicing modifier drug; in another embodiment, provided is a transcript of the nucleic acid molecule according to the present disclosure in the absence of a small-molecule alternative splicing modifier drug; wherein the the small-molecule alternative splicing modifier drug is LMI070, or a derivative having the same binding site as LMI070 on the alternative splicing modulatory element, or a pharmaceutically acceptable salt of LMI070; or the small-molecule alternative splicing modifier drug is Risdiplam, or a derivative having the same binding site as Risdiplam on the alternative splicing modulatory element, or a pharmaceutically acceptable salt of Risdiplam.

In one embodiment, the nucleic acid molecule is a DNA molecule and the transcript is an RNA molecule.

In one embodiment, the transcript is an mRNA comprising a first exon and a second exon sequentially from the 5' end to the 3' end, and the nucleotide sequence is SEQ ID NO:14, or comprises SEQ ID NO:14.

In another embodiment, the transcript is an mRNA comprising a first exon, a pseudoexon and a second exon sequentially from the 5' end to the 3' end, and the boundary sequence between the pseudoexon and the second exon is preferably TGATTT, wherein TGA belongs to the pseudoexon and TTT belongs to the second exon, and further the nucleotide sequence of the mRNA is SEQ ID NO: 15, or comprises SEQ ID NO: 15.

In a fourth aspect of the present disclosure, the present disclosure provides a vector comprising the nucleic acid molecule according to the present disclosure or the transcript according to the present disclosure.

In one embodiment, the vector is a DNA or RNA vector. Preferably, the vector is a circular vector. More preferably, the vector is a plasmid.

In one embodiment, the vector is double-stranded or single-stranded; preferably, the vector is double-stranded.

In one embodiment, the vector is a viral vector. Preferably, the viral vector is an adeno-associated virus (AAV) vector, a chimeric AAV vector, an adenoviral vector, a retroviral vector, a lentiviral vector, a herpes simplex virus vector, a baculoviral vector, or any mutant or derivative thereof. Preferably, the viral vector is a recombinant AAV vector, a self-complementary AAV (scAAV) vector, or a single-stranded AAV (ssAAV) vector. Preferably, the recombinant AAV vector comprises one or more inverted terminal repeats (ITRs), optionally wherein the ITRs are AAV2 ITRs, optionally wherein the AAV vector comprises two ITRs.

In the fifth aspect of the present disclosure, the present disclosure provides a recombinant virus, which comprises the alternative splicing modulatory element according to the first aspect of the present disclosure, the nucleic acid molecule according to the second aspect of the present disclosure, the transcript according to the third aspect of the present disclosure, or the vector according to the fourth aspect of the present disclosure.

In one embodiment, the recombinant virus is an adeno-associated virus (AAV), a chimeric AAV, an adenovirus, a retrovirus, a lentivirus, a herpes simplex virus, a baculovirus, or any mutant or derivative thereof. Preferably, the recombinant virus is AAV. More preferably, the AAV comprises one or more of the following: AAV1, AAV2, AAV3, AAV4, AAV5, AAV6, AAV7, AAV8, AAV9, AAV10, AAV11, AAV12, AAVrh8, AAVrh10, AAVrh36, AAVrh37, AAVrh74, AAVrh79, AAV-DJ, AAV-DJ/8, AAV.Anc80, AAV.Anc80L65, AAV-PHP.B, AAV-PHP.B2, AAV-PHP.B3, AAV-PHP.A, AAV-PHP.eB, AAV-PHP.S, AAV2i8, MyoAAV, AAVMYO, AAV.CPP.16 capsid serotypes or variants thereof, such as a combination of capsids from more than one AAV serotype. In one embodiment, the AAV is AAV9.

In the sixth aspect of the present disclosure, the present disclosure provides a cell, which comprises one or more alternative splicing modulatory elements as described in the first aspect of the present disclosure, or comprises one or more nucleic acid molecules as described in the second aspect of the present disclosure, or comprises one or more transcripts as described in the third aspect of the present disclosure, or comprises one or more vectors as described in the fourth aspect of the present disclosure, or comprises one or more recombinant viruses according to the fifth aspect of the present disclosure.

In one embodiment, the cell is a human cell. The human cells may be cells in vivo, or cells separated from the body; or immortalized cell lines or cancer cells.

Preferably, the human cell is a cardiac cell, a muscle cell, a neuron, a hepatocyte cell, a splenocyte cell, a lung cell or a renal cell.

In one embodiment, when a small-molecule alternative splicing modifier drug is present in the cell, the expression level of the target gene is 1 to 2000-fold higher than the expression level of the target gene when the a small-molecule alternative splicing modifier drug is not present in the cell, for example, 1 to 100-fold, 1 to 300-fold, 1 to 500-fold, 1 to 700-fold, 1 to 900-fold, 1 to 1100-fold, 1 to 1300-fold, 1 to 1500-fold, 1 to 1700-fold, 1 to 1900-fold, 1 to 2000-fold higher; optionally, when the alternative splicing modulating small molecule drug is not present in the cell, the expression level of the target gene cannot be detected.

In the seventh aspect of the present disclosure, a pharmaceutical composition is provided, which comprises one or more alternative splicing modulatory elements as described in the first aspect of the present disclosure, or one or more nucleic acid molecules as described in the second aspect of the present disclosure, or one or more transcripts as described in the third aspect of the present disclosure, or one or more vectors as described in the fourth aspect of the present disclosure, or one or more recombinant viruses according to the fifth aspect of the present disclosure, or one or more cells according to the sixth aspect of the present disclosure.

In an eighth aspect of the present disclosure, a method is provided for modulating the expression of a target gene based on an alternative splicing modulating small molecule drug, the method comprising: combining one or more alternative splicing modulating elements as described in the first aspect of the present disclosure, or one or more nucleic acid molecules as described in the second aspect of the present disclosure, or one or more transcripts as described in the third aspect of the present disclosure, or one or more vectors as described in the fourth aspect of the present disclosure, or one or more recombinant viruses as described in the fifth aspect of the present disclosure, or one or more cells as described in the sixth aspect of the present disclosure with one or more alternative splicing modulating small molecule drugs. The method comprises contacting a target gene with a substance, wherein, when the alternative splicing modulating small molecule drug is present, the expression level of the target gene is 1 to 2000-fold higher than the expression level of the target gene in the absence of the small-molecule alternative splicing modifier drug, for example, 1 to 100-fold, 1 to 300-fold, 1 to 500-fold, 1 to 700-fold, 1 to 900-fold, 1 to 1100-fold, 1 to 1300-fold, 1 to 1500-fold, 1 to 1700-fold, 1 to 1900-fold, 1 to 2000-fold higher; optionally, when the small-molecule alternative splicing modifier drug is not present, the expression level of the target gene cannot be detected.

In the ninth aspect of the present disclosure, a gene therapy method is provided for treating a subject in need, the gene therapy method comprising administering to the subject one or more alternative splicing modulatory elements as described in the first aspect of the present disclosure, or one or more nucleic acid molecules as described in the second aspect of the present disclosure, or one or more transcripts as described in the third aspect of the present disclosure, or one or more vectors as described in the fourth aspect of the present disclosure, or one or more recombinant viruses as described in the fifth aspect of the present disclosure, or one or more cells as described in the sixth aspect of the present disclosure, or one or more pharmaceutical compositions as described in the seventh aspect of the present disclosure, and one or more small-molecule alternative splicing modifier drugs; wherein, when the small-molecule alternative splicing modifier drug is present, the expression level of the target gene is 1 to 2000-fold higher than the expression level of the target gene in the absence of the small-molecule alternative splicing modifier drug, for example, 1 to 100-fold, 1 to 300-fold, 1 to 500-fold, 1 to 700-fold, 1 to 900-fold, 1 to 1100-fold, 1 to 1300-fold, 1 to 1500-fold, 1 to 1700-fold, 1 to 1900-fold, 1 to 2000-fold higher; optionally, when the small-molecule alternative splicing modifier drug is not present, the expression level of the target gene cannot be detected.

In the tenth aspect of the present disclosure, a kit is provided, the kit comprising: (1) one or more alternative splicing modulatory elements as described in the first aspect of the present disclosure, or one or more nucleic acid molecules as described in the second aspect of the present disclosure, or one or more transcripts as described in the third aspect of the present disclosure, or one or more vectors as described in the fourth aspect of the present disclosure, or one or more recombinant viruses as described in the fifth aspect of the present disclosure, or one or more cells as described in the sixth aspect of the present disclosure, or one or more pharmaceutical compositions as described in the seventh aspect of the present disclosure; and/or (2) one or more small-molecule alternative splicing modifier drugs.

In the eleventh aspect of the present disclosure, an application of (a) and (b) for modulating the expression level of a target gene or for preparing a gene therapy drug is provided, wherein (a) is selected from one or more of the alternative splicing modulatory elements as described in the first aspect of the present disclosure, or one or more of the nucleic acid molecules as described in the second aspect of the present disclosure, or one or more of the transcripts as described in the third aspect of the present disclosure, or one or more of the vectors as described in the fourth aspect of the present disclosure, or one or more of the recombinant viruses as described in the fifth aspect of the present disclosure, or one or more of the cells as described in the sixth aspect of the present disclosure, or one or more of the pharmaceutical compositions as described in the seventh aspect of the present disclosure, and (b) is a small-molecule alternative splicing modifier drug.

Preferably, the indications that the gene therapy drug can treat include heart diseases (such as ischemic heart disease and cardiomyopathy), muscular dystrophy, neuromuscular diseases, progeria, retinitis pigmentosa (also known as retinitis pigmentosa), Leber's congenital amaurosis (LCA), age-related macular degeneration, spinal muscular atrophy (SMA), hemophilia, etc.

In a twelfth aspect of the present disclosure, a probe is provided, wherein the probe targets the junction between the pseudoexon and the second exon in the transcript in the presence of a small-molecule alternative splicing modifier drug in the third aspect of the present disclosure.

In one embodiment, the probe comprises a nucleotide sequence having at least 95% identity to the nucleotide sequence of SEQ ID NO:16.

In one embodiment, the 5' end of the probe is modified with a fluorescent group, and the 3' end of the probe is modified with a non-fluorescent quenching group; wherein the fluorescent group includes but is not limited to HEX, VIC, FAM or TET, and the non-fluorescent quenching group includes but is not limited to MGB, TAMRA or BHQ.

In the thirteenth aspect of the present disclosure, a method is provided for detecting the alternative splicing modulatory element according to the first aspect of the present disclosure, or one or more nucleic acid molecules according to the second aspect of the present disclosure, or one or more transcripts according to the third aspect of the present disclosure, or one or more vectors according to the fourth aspect of the present disclosure, or one or more recombinant viruses according to the fifth aspect of the present disclosure, or one or more cells according to the sixth aspect of the present disclosure, or one or more pharmaceutical compositions according to the seventh aspect of the present disclosure, wherein the method comprises a qPCR amplification step, and the probe as described in the twelfth aspect of the present disclosure is used in the qPCR amplification step. The qPCR amplification step includes contacting the mRNA in the transcript as described in the present disclosure or its PCR amplification product with one or more probes as described in the present disclosure, and then quantitatively detecting the degree of reaction. When the probe carries a fluorescent group, the degree of reaction can be characterized by the intensity of the fluorescent signal.

In one embodiment, the forward primer used in the qPCR amplification step is selected from SEQ ID NO:17.

In one embodiment, the method further comprises an RT-PCR amplification step before the qPCR amplification step. Preferably, the forward primer used in the RT-PCR amplification step is selected from SEQ ID NO:18.

The beneficial effects achieved by the present disclosure include:
(1) The RNA splicing modulatory drug used in the prior art is limited to LMI070, while the alternative splicing modulatory elements provided by the present disclosure can be regulated by a variety of small-molecule alternative splicing modifier drugs, such as Risdiplam and LMI070. Among them, Risdiplam is a drug approved by the FDA for marketing and can be used for clinical treatment, while LMI070 has not yet been approved by the FDA for marketing. Therefore, the alternative splicing modulatory element provided in the present disclosure has greater application value and broader application prospects.
(2) The ability of RNA splicing modulatory drugs to regulate gene expression in the prior art is limited, usually not exceeding 200-fold, while the alternative splicing modulatory elements provided in the present disclosure can achieve a regulation up to 2000-fold.
(3) The existing methods for detecting changes in RNA splicing are limited. The present disclosure designs probes that can accurately detect RNA splicing for multiple alternative splicing modulatory elements with different compositions, and the disclosure also constructs an accurate detection method.
(4) The nucleic acid sequence in the prior art is relatively length, not less than 560 bp, and the intron sequence with key functions is unclear. The present disclosure conducts segment-by-segment truncation tests on the initially obtained alternative splicing modulatory elements, thereby identifying the key intron sequences therein, thereby obtaining alternative splicing modulatory elements with both low load and high modulatory ability. For example, starting from the alternative splicing modulatory element Zf1, after optimization, we obtained the Zf2 sequence and Zf3 sequence, which have similar functions to Zf1, among which the Zf3 sequence is only 403bp.
(5) The existing technology is derived from cells cultured in vitro, and it is not clear whether it can also work in organs such as the heart, skeletal muscle, and liver in vivo. The alternative splicing modulatory element sequence provided by the present disclosure is derived from cardiac tissue, and it has been determined that it has functions in organs such as the heart, skeletal muscle, and liver.
(6) Existing technologies will add extraneous amino acid sequences with unknown function to the downstream expressed target gene, and the technology for expressing the target gene seamlessly is still unclear. There are no extraneous sequences between the alternative splicing modulatory elements provided by the present disclosure and the downstream target gene, or there are sequences that only play a connecting role and have no expression function (such as P2A sequences), which removes the extraneous amino acids expressed by the alternative splicing modulatory elements of the prior art and achieves traceless target gene expression.

### BRIEF DESCRIPTION OF DRAWINGS

In order to more clearly illustrate the specific embodiments of the present disclosure or the technical solutions in the prior art, the drawings required for use in the specific embodiments or the description of the prior art will be briefly introduced below. Obviously, the drawings as described below are some embodiments of the present disclosure. For ordinary technicians in this field, other drawings can be obtained based on these drawings without paying any creative work.
Figure 1 shows the discovery process of alternative splicing fragments regulated by Risdiplam (RIS) in mouse heart;
Figure 2 is a process of modifying candidate sequences and screening out the gene expression modulatory switch Zfr;
Figure 3 is the in vitro validation and evaluation results of the gene expression modulatory switch Zfr;
Figure 4 is the in vivo validation and evaluation results of the gene expression modulatory switch Zfr;
Figure 5 is a comparative analysis of LMI070 (LMI) and RIS acting on X^{on} or Zfr, respectively;
Figure 6 is the study and verification of Zfr controlling YAP gene expression;
Figure 7 is the research and verification of Zfr controlling CRISPR/Cas9 gene editing;
Figure 8 shows a study on reducing the size of the Zf element by deleting part of the intron sequence.

### DETAILED DESCRIPTION OF THE EMBODIMENTS

### (I) Definitions or terms

The following abbreviations are used for the relevant definitions or terms involved in this disclosure. Unless defined otherwise, all technical and scientific terms used herein have the meanings commonly understood by one of ordinary skill in the art. The following terms are provided below.

As used herein, the singular forms "a", "an" and "the" include plural forms unless the context clearly indicates otherwise. As will be apparent to the skilled artisan from the teachings contained herein, the terms "about" or "approximately" when used in the context of numerical values and ranges refer to a value or range that is approximately or close to the specified value or range such that the embodiments may perform as intended. In some embodiments, "about" means ±10% of the numerical amount.

The terms "polynucleotide" and "nucleic acid" are used interchangeably herein and refer to a polymeric form of nucleotides of any length. They may include one or more ribonucleotides or deoxyribonucleotides. Thus, the term includes, but is not limited to, single-stranded, double-stranded, or multi-stranded DNA or RNA, genomic DNA, cDNA, DNA-RNA hybrids, or polymers comprising purine and pyrimidine bases or other natural, chemically or biochemically modified, non-natural or derivatized nucleotide bases, such as locked nucleic acids (LNA), peptide nucleic acids (PNA).

The terms "peptide", "polypeptide" and "protein" are used interchangeably to refer to a compound composed of amino acid residues covalently linked by peptide bonds. A protein or peptide typically comprises at least two amino acids or amino acid variants, and there is no limit to the maximum number of amino acids that may comprise a protein or peptide sequence. Polypeptides include any peptide or protein comprising two or more amino acids or variants linked to each other by peptide bonds. These terms include, for example, biologically active fragments, substantially homologous polypeptides, oligopeptides, homodimers, heterodimers, variants of polypeptides, modified polypeptides, derivatives, analogs, fusion proteins. The polypeptides include natural peptides, recombinant peptides, or a combination thereof.

The terms "identity" and "homology" are used interchangeably herein and when used to describe polynucleotide or polypeptide sequences refer to the percentage of bases or amino acids that are identical and in the same relative position when two sequences of polypeptides or polynucleotides are compared or aligned. Sequence identity can be determined in a number of different ways. For example, sequences can be aligned via various methods and computer programs (e.g., BLAST, T-COFFEE, MUSCLE, MAFFT, etc.).

A nucleic acid molecule described in the present disclosure can be, for example, an isolated nucleic acid molecule. The term "isolated" when applied to nucleic acid or protein molecules refers to a nucleic acid or protein that has been separated from one or more components with which it is normally associated in its natural environment. Separation can include separation from a larger nucleic acid (e.g., from a gene or chromosome) or from other proteins or molecules with which the nucleic acid or protein is normally in contact. The term encompasses but does not require complete separation.

The nucleic acid molecules provided by the present disclosure comprise an alternative splicing modulatory element and a target gene encoding a target molecule (e.g., a target protein), wherein the target gene is operably linked to the 3' end of the alternative splicing modulatory element. Those skilled in the art will understand that the connection between the target gene and the alternative splicing modulatory element can be a tight connection, that is, there is no nucleic acid sequence between the target gene and the alternative splicing modulatory element; the connection between the target gene and the alternative splicing modulatory element can also be a non-tight connection, that is, there are other nucleic acid sequences between the target gene and the alternative splicing modulatory element, as long as the other nucleic acid sequences do not affect the expression of the target gene and do not affect the function of the alternative splicing modulatory element, the purpose of the present disclosure can be met, for example, the other nucleic acid sequence does not contain a stop codon or a start codon (such as ATG), and the number of nucleotides of the other nucleic acid sequence is a multiple of 3, so as not to affect the reading frame of the target gene.

The nucleic acid molecule provided by the present disclosure may be any type of nucleic acid molecule, as long as the nucleic acid molecule can achieve the purpose of the present disclosure. For example, the nucleic acid molecule of the present disclosure can be a single-stranded or double-stranded deoxyribonucleic acid molecule (i.e., a DNA molecule), or a single-stranded or double-stranded ribonucleic acid molecule (i.e., an RNA molecule); the nucleic acid molecule of the present disclosure can be linear or circular.

The term "operably linked" refers to a functional relationship between two or more polynucleotide (e.g., DNA) segments. Typically, the term refers to the functional relationship between a transcriptional modulatory sequence and a sequence to be transcribed. For example, in an appropriate host cell or other expression system, a promoter or an enhancer sequence is operably linked to a coding sequence if it stimulates or modulates the transcription of the coding sequence. Typically, promoter transcriptional modulatory sequences operably linked to a sequence are contiguous with the sequence or separated by a short spacer sequence, that is, they are cis-acting. However, some transcriptional modulatory sequences, such as enhancers, need not be physically contiguous or positioned in close proximity to the coding sequences whose transcription they enhance.

The term "DreAM" is an abbreviation for Drug-elicitable Alternative-splicing Modulator, which is a nucleic acid molecule that regulates gene expression using RNA splicing modulators.

Various exemplary embodiments of the present disclosure are described in detail below with reference to the accompanying drawings. The description of the exemplary embodiments is merely illustrative and is not intended to limit the invention, its application, or uses. The present disclosure may be embodied in many different forms and is not limited to the embodiments set forth herein. These embodiments are provided so that this disclosure will be thorough and complete, and will fully convey the scope of the disclosure to those skilled in art. It should be noted that unless otherwise specifically stated, the technical means described in these embodiments should be interpreted as merely illustrative rather than restrictive.

### Alternative splicing modulatory element

The alternative splicing modulatory element provided by the present disclosure comprises a plurality of introns and exons and a nucleic acid sequence harboring a binding site for a small-molecule alternative splicing modifier drug. In an embodiment, the alternative splicing modulatory element is operably linked to the target gene, and the target gene is positioned downstream of the alternative splicing modulatory element. The alternative splicing modulatory element provided by the present disclosure is used in combination with one or more small-molecule alternative splicing modifier drugs, to regulate (e.g., turn on) the expression of the target gene. The regulation based on the combined use of alternative splicing modulatory elements and one or more small-molecule alternative splicing modifier drugs provided by the present disclosure shows a dose-dependency, that is, the expression level of the target gene can be controlled by administering different doses of the small-molecule alternative splicing modifier drug. In addition, the regulation based on the combined use of alternative splicing modulatory elements and one or more small-molecule alternative splicing modifier drugs provided by the present disclosure presents a time correlation, that is, the expression level of the target gene can be controlled by administering the small-molecule alternative splicing modifier drugs at different-fold.

In various aspects, the alternative splicing modulatory element comprises at least, in order from the 5' to the 3' end: a first exon, a first intron, a pseudoexon, a second intron, and a second exon. One or more small-molecule alternative splicing modifier drugs bind to the sequence at the junction of the pseudoexon and the second intron. Wherein, in the presence of the small-molecule alternative splicing modifier drug, the pseudoexon is included in the mRNA product of the nucleic acid; and in the absence of the small-molecule alternative splicing modifier drug, the pseudoexon is not included in the mRNA product of the nucleic acid.

In the present disclosure, the pseudoexon of the alternative splicing modulatory element has a sole start codon (e.g., ATG sequence).

### Small-molecule alternative splicing modifier drug

The small-molecule alternative splicing modifier drug used in the present disclosure refers to a compound that can modify alternative splicing. In some embodiments, the small-molecule alternative splicing modifier drug regulates (particularly increases) pseudoexon inclusion. In some embodiments, the small-molecule alternative splicing modifier drug binds to a sequence at a specific position in the alternative splicing modulatory element. Preferably, in some embodiments, the specific position in the alternative splicing modulatory element to which the small-molecule alternative splicing modifier drug binds is positioned at the junction of the 3' end of the pseudoexon and the 5' end of the second exon. More preferably, in some embodiments, the specific position in the alternative splicing modulatory element to which the small-molecule alternative splicing modifier drug binds is positioned at the AGAGTA sequence at the junction of the 3' end of the pseudoexon and the 5' end of the second exon, wherein AGA is the last 3 nucleotide residues at the 3' end of the pseudoexon, and GTA is the 1st to 3rd nucleotide residues at the 5' end of the second intron.

In the first aspect of the small-molecule alternative splicing modifier drug, small-molecule alternative splicing modifier drug used in the present disclosure is LMI070 or a derivative having the same binding site as LMI070 on the alternative splicing modulatory element or a pharmaceutically acceptable salt of LMI070, wherein LMI070, also known as Branaplam or NVS-SM1, is a compound having a structure of the following formula (1).

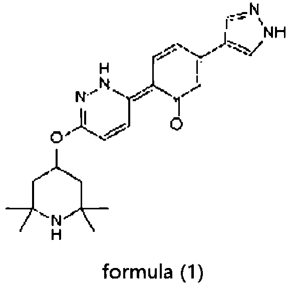

The derivative having the same binding site as LMI070 on the alternative splicing modulatory element refers to a derivative compound obtained by replacing the group conventionally used in the art on the basis of the compound of formula (1), and the derivative compound binds to the same sequence fragment as LMI070 on the alternative splicing modulatory element; as a means of testing whether it belongs to the derivative defined in the present disclosure, those skilled in the art can determine it through simple experiments, for example, whether the alternative splicing modulatory element as described in the present disclosure can also produce the same effect as LMI070, that is, if the addition of a certain derivative of LMI070 can also promote pseudoexon inclusion in mRNA, then this kind of derivative belongs to the derivative having the same binding site as LMI070 on the alternative splicing modulatory element as described in the present disclosure.

Examples of pharmaceutically acceptable salts of LMI070 include, but are not limited to, inorganic and organic acid salts, such as hydrochloride, hydrobromide, sulfate, citrate, lactate, tartrate, maleate, fumarate, mandelate and oxalate; and inorganic and organic base salts formed with bases such as sodium hydroxy, tris(hydroxymethyl)aminomethane (TRIS, tromethamine) and N-methylglucamine.

In the second aspect of the small-molecule alternative splicing modifier drug, the small-molecule alternative splicing modifier drug used in the present disclosure is Risdiplam or a derivative having the same binding site as Risdiplam on the alternative splicing modulatory element or a pharmaceutically acceptable salt of Risdiplam, wherein Risdiplam is a compound having a structure of the following formula (2).

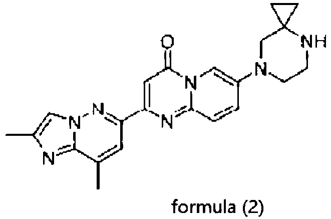

The derivative having the same binding site as Risdiplam on the alternative splicing modulatory element refers to a derivative compound obtained by replacing the group conventionally used in the art on the basis of the compound of formula (2), and the derivative compound binds to the same sequence fragment as Risdiplam on the alternative splicing modulatory element; as a means of testing whether it belongs to the derivative defined in the present disclosure, those skilled in the art can determine it through simple experiments, for example, whether the alternative splicing modulatory element as described in the present disclosure can also produce the same effect as Risdiplam, that is, if treatment with derivative of Risdiplam can also promote the pseudoexon inclusion in mRNA, then this kind of derivative belongs to the derivative having the same binding site as Risdiplam on the alternative splicing modulatory element as described in the present disclosure.

Examples of pharmaceutically acceptable salts of risdiplam include, but are not limited to, inorganic and organic acid salts, such as hydrochloride, hydrobromide, sulfate, citrate, lactate, tartrate, maleate, fumarate, mandelate and oxalate; and inorganic and organic base salts formed with bases such as sodium hydroxybutyrate, tris(hydroxymethyl)aminomethane (TRIS, tromethamine) and N-methylglucamine.

### Cleavage site

In some embodiments, the nucleic acid molecules of the present disclosure optionally include one or more sequences encoding cleavage sites, wherein the sequences encoding cleavage sites are positioned between the alternative splicing modulatory element and the target gene, so as to achieve the purpose of separating the alternative splicing modulatory element and the target gene. The cleavage site may be a self-cleavage site, a protease cleavage site, or a combination of both.

### 2A peptide

2A peptides are short peptides (about 18~25 amino acids) derived from viruses. They are often called "self-cleaving" peptides and can enable a single transcription product to produce multiple proteins. The 2A peptide is not completely "self-cleaving" but works by causing the ribosome to skip the synthesis of the glycine and proline peptide bonds at the C-terminus of the 2A element, ultimately leading to the separation of the end of the 2A sequence and the downstream product. Among them, some additional 2A residues will be added to the C-terminus of the upstream protein, while the N-terminus of the downstream protein will have additional proline. Currently, there are four commonly used 2A peptides, namely P2A, T2A, E2A and F2A, which come from four different viruses.

### Target gene

The expression of the target genes is controlled by the alternative splicing modulatory elements provided in the present disclosure. The target gene is a gene encoding a protein or nucleic acid whose expression is desired to be increased, decreased or controlled in vivo or in cells for gene therapy or other purposes. Without theoretical limitations, a skilled artisan can use the alternative splicing modulatory elements disclosed herein to construct a nucleic acid molecule comprising any gene of interest. In some embodiments, the gene of interest may be a protein-coding gene, such as a gene encoding an antibody or a functional binding fragment, a receptor, an enzyme, or the like. In some embodiments, the gene of interest can be a gene encoding a transcription activator-like effector nuclease (TALEN), a zinc finger nuclease (ZFN), or a Cas9 protein. In some embodiments, the target gene can be a gene that is transcribed to produce RNA, such as a gene that is transcribed to produce inhibitory RNA, such as miRNA, shRNA, LncRNA, etc. The number of target genes contained in the nucleic acid molecules provided by the present disclosure may be more than one or more.

### Detection of nucleic acid molecules containing alternative splicing modulatory elements

The nucleic acid molecules provided by the present disclosure include alternative splicing modulatory elements and target genes, wherein the composition of the alternative splicing modulatory elements has restrictive conditions as described in the present disclosure, but those skilled in the art can understand that the selection range of target genes is broad and not fixed, and different target genes can be selected according to the different purposes of using the nucleic acid molecules of the present disclosure.

Based on this, for the purpose of qualitative or quantitative detection of the presence of the nucleic acid molecules as described in the present disclosure, a method such as RT-PCR can be used for detection, wherein the forward primer of the primer pair used is designed according to the alternative splicing modulatory element, but the reverse primer needs to be designed according to the different target genes. The principles, tools and methods of primer design known in the art can be used to design and screen reverse primers.

### Fluorescent probes

A fluorescent probe (e.g., a TaqMan probe) is an oligonucleotide with an emitting group and a quenching group labeled at both ends, respectively. When the probe is intact, the fluorescent signal emitted by the emitting group is absorbed by the quenching group; during PCR amplification, the 5' to 3' exonuclease activity of the Taq enzyme cleaves and degrades the probe, separating the fluorescent emitting group and the fluorescent quenching group, so that the fluorescent monitoring system can receive the fluorescent signal of the fluorescent emitting group, that is, every time a DNA chain is amplified, a fluorescent molecule is formed, achieving complete synchronization between the accumulation of the fluorescent signal and the formation of the PCR product, thereby achieving quantification. Currently, commonly used fluorescent emitting groups include FAM, TET, VIC, HEX, etc., and commonly used fluorescence quenching groups include MGB, TAMRA, BHQ, etc.

### Zfr gene

Zfr encodes zinc finger RNA binding protein, which encodes an RNA binding protein characterized by its DZF (domain associated with zinc fingers) domain. The encoded protein may play a role in the nucleocytoplasmic shuttling of Staufen homolog 2, another RNA-binding protein in neurons. The expression of this gene is regulated by alternative polyadenylation mediated by differential microRNA targeting. Elevated expression of this gene has been observed in human patients with pancreatic cancer, and knockdown of this gene may result in reduced viability and invasiveness of pancreatic cancer cells.

### Tlk2 gene

TLK2 (Tousled-like kinase 2) is a protein-coding gene. Disorders associated with TLK2 include autosomal dominant type 57 intellectual disability and pica. Related pathways include the regulation of DNA damage response by miRNAs and the role of Chks in checkpoint regulation.

### Agrn gene

AGRN (Agrin) is a protein-coding gene. Diseases associated with AGRN include myasthenic syndrome congenital8and presynaptic congenital myasthenic syndrome. The relevant pathways include chondroitin sulfate/dermatan sulfate metabolism and glycosaminoglycan metabolism.

### Ubap2l gene

UBAP2L (ubiquitin-associated protein 2-like) is a protein-coding gene. Diseases associated with UBAP2L include spinal neuroblastoma and spinal primitive neuroectodermal tumors. Its related pathways include VEGFA-VEGFR2 signaling.

### Angel2 gene

ANGEL2 (Angel homolog gene 2) is a protein-coding gene. Diseases associated with ANGEL2 include pontine cerebellar dysplasia, type 7 and rheumatoid arthritis-associated interstitial lung disease.

### E030003E18Rik gene

It originates from 20495496-20495544 of mouse chromosome 19 (NCBI Gene ID: 320092). Currently, there is no related research report on this gene.

### (II) Exemplary Embodiments

The following embodiments illustrate the technical solutions of the present disclosure, but do not constitute any limitation to the protection scope of the present disclosure. The wild-type C57BL/6 mice was used as an animal model in animal experiments related to the present disclosure. Animal production, breeding, and experiments were performed at the Department of Laboratory Animal Science, Peking University Health Science Center. The relevant experimental operations involved were approved by the Biomedical Ethics Committee of Peking University (approval number: 2022412).

### Example 1 Discovery of splicing modulatory elements responsive to Risdiplam

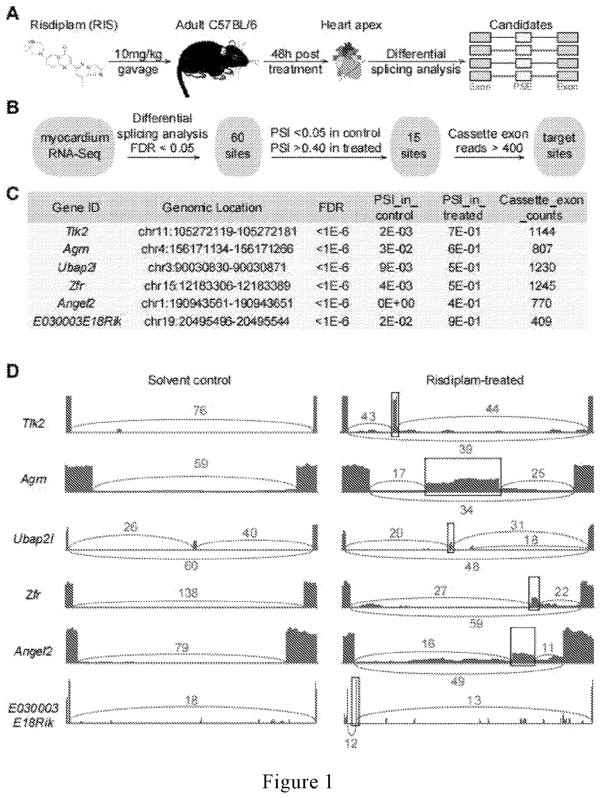

The process of discovering alternative splicing segments regulated by Risdiplam in mouse heart was shown in Figure 1A. Risdiplam (T16757, Target Mol) was selected as a small molecule splicing modulator and stored as a powdered solid in a -80°C refrigerator for long-term use. Before each experiment, it was prepared into a 1 mg/ml solution for animal administration. The solvent formula of the solution is 10% DMSO + 40% PEG300 + 5% TWEEN 80 + 45% physiological saline.

The mice were weighed and administered at a dose of 10 mg/kg. Small molecule splicing modulators were administered to adult mice aged 6 to 10 weeks by gavage while they were awake. The control group mice were given an equal amount of solvent (SOL). n=5 per group. 48 hours after administration, the mice were anesthetized with isoflurane and sacrificed, and the apical tissue was quickly obtained and frozen at -80°C for RNA sequencing experiments.

RNA was extracted by the following method: firstly, mouse tissues were broken using a homogenizer (DREMEL F6/10), and then RNA was extracted using Trizol (TransGen, ER501-01), aliquoted and stored at -80°C. After the extracted RNA quality inspection, 1 mg of reverse transcribed cDNA was taken, and the bulk transcriptome library was constructed using the KAPA Hyper Prep Kits library construction kit (KAPA, KK8504). After quality inspection by Fragment Analyzer 1.0.2.9 and qPCR quantification, PE150 double-end sequencing (next-generation sequencing) was performed using the Illumina Nova-seq sequencing platform, and the returned sequencing data was analyzed using bioinformatics technology.

The results of above-mentioned next-generation sequencing were preprocessed using Trim Galore software, and the processed data were quality controlled using FastQC software. The quality-controlled sequencing results were then aligned with the mouse mm10 reference genome using STAR software and screened based on the statistical analysis results. Then, CASH software was used for alternative splicing analysis, yielding 6 potential alternative splicing candidate sites (including Tlk2, Agrn, Ubap2l, Zfr, Angel2 and E030003E18Rik gene sites, B to C in Figure 1, where C shows the location of the alternative sequence in the mouse genome, including the gene name containing the sequence, and the specific coordinates in the mouse mm10 reference genome). The screening thresholds included: False Discovery Rate (FDR) < 0.05; in the solvent control group, Percent Spliced-In (PSI) < 0.05; in the RIS treatment group, Percent Spliced-In (PSI) > 0.4; the alternative splicing type was "cassette exon"; the number of reads related to alternative splicing in RNA-seq data is >400; and ensure the alternative splicing fragments comprising pseudoexons (D in Figure 1, sashimi diagram of alternative sequence RNA splicing, arcs and numbers indicate the number of reads of the sequencing sequence spanning the exon, and the black box marks the pseudoexon (PSE)).

### Example 2 Screening and obtaining Zfr splicing modulatory elements

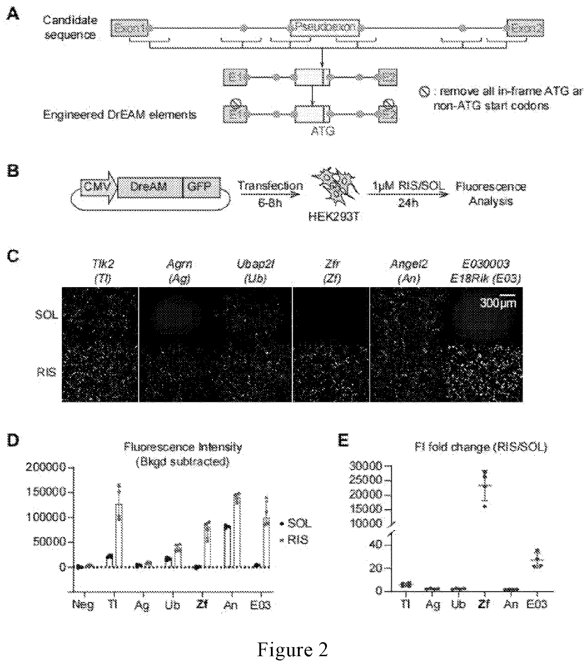

In order to obtain the optimal splicing modulatory element that responds to Risdiplam, it is necessary to modify the sequence fragment of the gene locus and perform in vitro verification and comparison on the potential alternative splicing candidate sites in Example 1:
The inventors retained the essential sequences for mRNA splicing at each gene locus (as shown by the dot positions marked in A in Figure 2 ), including the first exon-first intron junction sequence, the branch point sequence required for splicing in the first intron, the first intron-pseudoexon junction sequence, the pseudoexon-second intron junction sequence, the branch point sequence required for splicing in the second intron, and the second intron-second exon junction sequence. At the same time, a unique ATG was set into each pseudoexon as the start codon, the start codon sequences (such as ATG and CTG) in other components were deleted, and other non-essential sequences were deleted to reduce the volume for in vitro verification.

To validate the function of each engineering splicing modulatory element sequence from different genes, the inventors constructed a series of CMV-DreAM-GFP reporter plasmids (as shown in B of Figure 2). These plasmids were based on the plasmid AAV-U6sgRNA-U6sgRNA-Tnnt2-Cre, originally reported in their previous publication (Guo Y et al., Analysis of Cardiac Myocyte Maturation Using CASAAV, a Platform for Rapid Dissection of Cardiac Myocyte Gene Function In Vivo. Based on the AAV-U6sgRNA-U6sgRNA-Tnnt2-Cre plasmid vector disclosed in Circ Res., 120(12):1874-1888, 2017). In these plasmids, DreAM elements correspond to the engineered start/initial splicing modulatory elements derived from different genomic loci and are named DreAM-Tl (Tlk2), DreAM-Ag (Agrn), DreAM-Ub (Ubap2l), DreAM-Zf1 (Zfr), DreAM-An (Angel2), and DreAM-E03 (E030003E18Rik), and the nucleotide sequences are of SEQ ID NOs: 33-35, 10, and 36-37, respectively. Among these plasmids, CMV was a constitutive expression promoter, the start codon ATG of GFP was deleted and GFP was in the same reading frame as the ATG in DreAM-Tl/DreAM-Ag/DreAM-Ub/DreAM-Zf1/DreAM-An/DreAM-E03. At the same time, as a positive control group (PC), the inventors constructed a CMV-GFP reporter plasmid, in which the ATG of GFP gene was retained, and as a negative control group (NC), a CMV-(ATG)GFP reporter plasmid was constructed, in which the ATG of GFP gene was removed.

To screen and obtain the optimal splicing modulatory elements that respond to Risdiplam, the inventors transfected HEK293T cells with reporter plasmids CMV-DreAM-Tl-GFP, CMV-DreAM-Ag-GFP, CMV-DreAM-Ub-GFP, CMV-DreAM-Zf1-GFP, CMV-DreAM-An-GFP, and CMV-DreAM-E03-GFP. The cellstransfected with CMV-(ATG)GFP plasmid served as the negative control group. After 24 hours of treatment with 1 µM Risdiplam, the cell fluorescence signal was captured using a fluorescence microscope, and the fluorescence signal intensity was quantitatively analyzed (B to D in Figure 2, where C is the GFP fluorescence microscopy image of each group, and D is the quantification of GFP fluorescence intensity using a microplate reader (n=4)). The results showed that after the treatment of Risdiplam, the cells transfected with the splicing element reporter plasmid expressed GFP fluorescence signals, and Risdiplam has the function of inducing alternative splicing regulation. Moreover, the expression difference of GFP fluorescence signal of DreAM-Zf element exhibited the most significant before and after drug administration (E in Figure 2, the fluorescence intensity multiple of the RIS treatment relative to the SOL control group).

The starting/initial DreAM-Zf1 splicing modulatory sequence discovered in the present disclosure has a length of 1234bp, including the first exon-first intron connection sequence (CAGGTA), the branch point sequence required for splicing in the first intron (CTTAACAT, the fifth position A is the branch site), the first intron-pseudoexon connection sequence (TAGGTT), the pseudoexon-second intron connection sequence (ATGAGTA), the branch point sequence required for splicing in the second intron (TCTAACTC, the fifth position A is the branch site), and the second intron-second exon connection sequence (CAGTTT). The nucleotide sequence of the starting/initial DreAM-Zf1 is as follows:

ACTGACCAACAGCGTGAAGACATTACATCCAGTGCACAGGTAATTGAATTCTT
GTTGGATTACTGTCTCTAAGGCCTGGGAGTTTTATAATTGTTTCTTTATGTGTAATAA
TAACCGTACTAGTCATTGTGATACTTATTTGGGAAGTAAGCCTTTAAAGAGGTTATT
AAAAAGATTTACTGATTCCCACACCGGAAGTTGCTCTTCTAGCCGTGAAAGAAGGA
CGGCGTGGTCTTCAGTTTGCTATGTCCACTAACAATATGTCCGACCCACGAAGGCCC
ACCAAAGTTATACTTGTGGCACATATAAACCTTTGGAATCAAGGGACCTGATACTG
GATTATTAAATATTTAAGAGAATTTATATTTAATAAACTTGATACTTCAGTGAGAAA
GGACAAATCATCAGTAGAATGGTTTTGAGATAGCTGATGATTCTATTAAAAGTAGA
TATGTGTGTGGGTTAAATGACTTAAGCATACATCAAAAGACTGAGAAAGGCTCTTA
ACATTAAAAGATGGGCCtttttttttCTTTTAGGTTAAAGGATTACATATTAAAAATATTGT
ATAGACTCTTTAAGGCCAAAGAAGTGTGCTCATTTCTCCCGATTAAAAGTTATGAGT
AAGAATTGGGTATTTTGCCTTTTGGGGGTGGGAGAtttgaaacagggtttctccttacagctctggctttcc
tggagcagtgaattgagcctggctcctctgtaggagtaacgagttgctttaaccactgacatctttccaccaccaccaccaccCCTTA

TGAATAGACTAAATTTATTTAAAGATTGATATTTTCTAGCTTAATTATTTGGAAAGC
AAATAAGCACACAGAAAAGCCTCCTCCCaaaaaatataaataaaaaaGTCTATCAGGACAT
AGTATATGTCCCTTCTGATTAATCATTGGTGGTGAAACAACCCATTCAGGGTGTCTC
TTCCATCACAGTGGAtttttttCATATTGTTACATGGAGAAGTTAACTTAGTAAGAATTG
ATGTGCTTCTGTGCCAGTGAGATAGCTCTGCAGGTAAAAGGGAGACTGACCCCCAA
CTTGTACTCTGGCCGTGGCAGTCATGTGCACACAAGTAAACTGAATAAATATAAAG
AGCAGATTTTAGAGTGTTTTGGCTTTAAATTAATTCTATATTCTAACTCTATTTGCAG
TTTGCATTGAGACTCCTTGCATTCCGTCAGATACACAAAGTT(Zf1, SEQ ID NO:10).

Among them, the division of each region of the alternative splicing modulatory element is of Table 1.

**Table 1 Sequences of various regions of alternative splicing modulatory elements**

| Regions | Sequences(5'→3') | Sequence number |
|---|---|---|
| first exon | ACTGACCAACAGCGTGAAGACATTAC ATCCAGTGCACAG | SEQ ID NO:2 |
| first intron | GTAATTGAATTCTTGTTGGATTACTGT CTCTAAGGCCTGGGAGTTTTATAATTGTTT CTTTATGTGTAATAATAACCGTACTAGTCA TTGTGATACTTATTTGGGAAGTAAGCCTTT AAAGAGGTTATTAAAAAGATTTACTGATT CCCACACCGGAAGTTGCTCTTCTAGCCGT GAAAGAAGGACGGCGTGGTCTTCAGTTTG CTATGTCCACTAACAATATGTCCGACCCA CGAAGGCCCACCAAAGTTATACTTGTGGC ACATATAAACCTTTGGAATCAAGGGACCT GATACTGGATTATTAAATATTTAAGAGAA TTTATATTTAATAAACTTGATACTTCAGTG AGAAAGGACAAATCATCAGTAGAATGGTT TTGAGATAGCTGATGATTCTATTAAAAGT AGATATGTGTGTGGGTTAAATGACTTAAG | SEQ ID NO:3 |
| | CATACATCAAAAGACTGAGAAAGGCTCTT AACATTAAAAGATGGGCCtttttttttCTTTTAG | |
| pseud oexon | GTTAAAGGATTACATATTAAAAATAT TGTATAGACTCTTTAAGGCCAAAGAAGTG TGCTCATTTCTCCCGATTAAAAGTTATGA | SEQ ID NO:1 |
| second intron | GTAAGAATTGGGTATTTTGCCTTTTGG GGGTGGGAGAtttgaaacagggtttctccttacagctctggc tttcctggagcagtgaattgagcctggctcctctgtaggagtaacga gttgctttaaccactgacatctttccaccaccaccaccaccCCTT ATGAATAGACTAAATTTATTTAAAGATTG ATATTTTCTAGCTTAATTATTTGGAAAGCA AATAAGCACACAGAAAAGCCTCCTCCCCaa aaaaatataaataaaaaaGTCTATCAGGACATAGTA TATGTCCCTTCTGATTAATCATTGGTGGTG AAACAACCCATTCAGGGTGTCTCTTCCAT CACAGTGGAtttttttCATATTGTTACATGGAG AAGTTAACTTAGTAAGAATTGATGTGCTT CTGTGCCAGTGAGATAGCTCTGCAGGTAA AAGGGAGACTGACCCCCAACTTGTACTCT GGCCGTGGCAGTCATGTGCACACAAGTAA ACTGAATAAATATAAAGAGCAGATTTTAG AGTGTTTTGGCTTTAAATTAATTCTATATT CTAACTCTATTTGCAG | SEQ ID NO:6 |
| second exon | TTTGCATTGAGACTCCTTGCATTCCGT CAGATACACAAAGTT | SEQ ID NO:9 |

| | | |
|---|---|---|
| Note: The sequence TGAGTA (the underlined sequence in this table) immediately adjacent to the 3' end of the pseudoexon and the 5' end of the second intron is the binding site of Risdiplam. | | |

### Example 3 Construction of a plasmid containing a Zfr splicing modulatory element

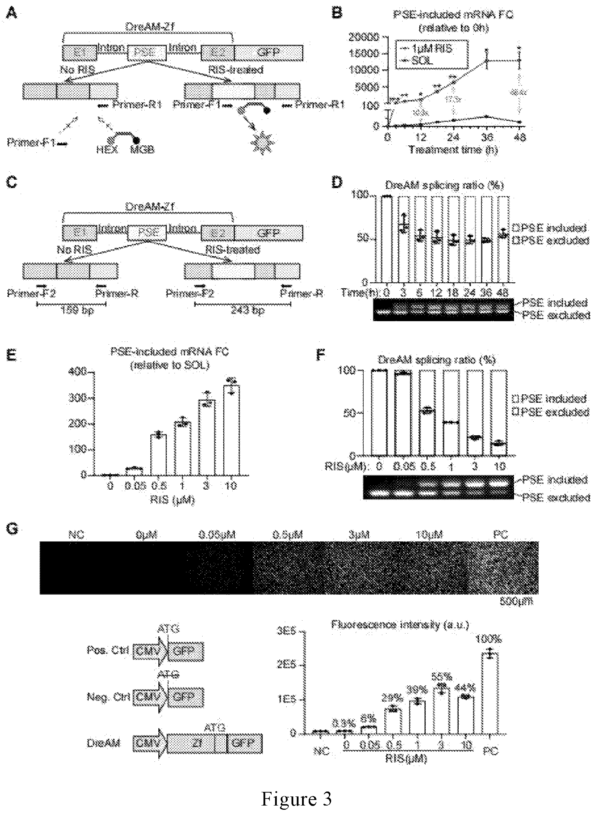

In order to accurately quantify the mRNA comprising pseudoexons regulated by alternative splicing modulatory elements, the inventors designed a TaqMan probe qPCR assay for detection, wherein the forward primer is designed to target the pseudoexon, the TaqMan probe is designed to target post-splicing junction sequence of the pseudoexon and the second exon, the emission group of probe is HEX and the quenching group is MGB, and the reverse primer targets the target gene (GFP) (A in Figure 3). HEK293T cells were transfected with CMV-DreAM-Zf1-GFP plasmid and then treated with 1 µM Risdiplam. DMSO was used as the solvent control group. After 0, 3, 6, 12, 18, 24, 36, and 48 hours, the cells were collected for TaqMan-qPCR detection and quantitative analysis (B in Figure 3, n = 3 for each group, t-test to compare the difference between RIS and SOL, *P<0.05, **P<0.01, ***P<0.001. Gray values mark the multiple of RIS versus SOL at each time point). The results showed that Risdiplam has the function of changing the splicing products of DreAM and enhancing pseudoexons inclusion in mRNA, and this function shows a positive correlation with the administration time. In order to further verify the alternative splicing regulation of nucleic acid modulatory elements, the inventors also performed RT-PCR analysis. The forward primer of RT-PCR was designed to target the first exon with sequence: CGTGAAGACATTACATCCAGTGCA (SEQ ID NO: 18); the reverse primer was designed to target the target gene (C in Figure 3) with sequence: TGAACTTGTGGCCGTTTACGT (SEQ ID NO: 42), which was used to quantify the relative mRNA abundance of containing PSE (243 bp band) and not containing PSE (159 bp band). The results showed that the function of Risdiplam inducing alternative splicing regulation took effect 3 hours after drug addition and tended to be saturated at 6 hours (D in Figure 3, the bottom is the RT-PCR electrophoresis diagram, and the upper part is the quantitative diagram (n=3)).

In order to explore the relationship between the Risdiplam administration concentration and the alternative splicing regulation of nucleic acid modulatory elements, the inventors transfected HEK293T cells with CMV-DreAM-Zf1-GFP plasmid, and then treated them with 0, 0.05, 0.5, 3, or 10 µM Risdiplam, respectively. The cells transfected with CMV-GFP plasmid were used as the positive control group, and the cells transfected with CMV-(ATG)GFP plasmid were used as the negative control group. After 24 hours of Risdiplam treatment, the fluorescence signal intensity was observed and quantitatively analyzed. At the same time, the cells were collected, and the pseudoexons inclusions and alternative splicing events mediated by Risdiplam-induced nucleic acid modulatory elements were quantified via TaqMan-qPCR and RT-PCR assays (E to G in Figure 3, in E and F, the cells are treated with different concentrations of RIS, and samples were collected 24 hours after treatment. The relationship between RIS concentration and RNA splicing was analyzed by qPCR (E) and RT-PCR (F). G is the quantitative analysis of GFP fluorescence signal after treatment with different concentrations of RIS for 24 h. The conventional reporter gene plasmid without DreAM-Zf1 was used as positive control. The values on the bar graph reflect the relative fluorescence intensity relative to the positive control). The results showed that after Risdiplam treatment, the cells expressed GFP fluorescence signals. Risdiplam has the function of inducing alternative splicing regulation, and this function shows a positive correlation with the dosage concentration.

### Example 4 Construction of AAV vector controlled by Zf1 element

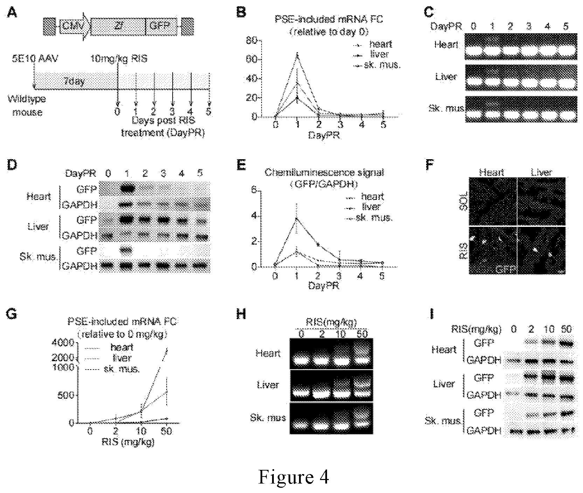

To verify whether the DreAM-Zf1 element is effective in vivo, the inventors packaged an AAV9 viral vector containing CMV-DreAM-Zf1-GFP. Among them, the method for constructing the AAV plasmid is as follows: the DreAM-Zf1 sequence is redesigned on the basis of retaining part of the wild-type mouse genome sequence, and primers were designed to amplify multiple fragments from mouse genomic DNA, and then these fragments were assembled through seamless cloning, as shown in Figure 4A. CMV and GFP sequences were obtained from public data sources such as Addgene. DNA fragments assembly and plasmid construction were performed by seamless cloning reactions.

The AAV packaging method is as follows: the plasmids used for AAV packaging are 70 µg AAV plasmid (containing CMV-DreAM-Zf1-GFP element), 70 µg AAV9 Rep/Cap plasmid and 160 µg pHelper helper plasmid. These plasmids were co-transfected into HEK293T cells cultured in 15 cm dishes using PEI transfection reagent (Yeasen, 40816ES03). 60 hours after transfection, the cells were scraped and resuspended in lysis buffer (20 mM Tris pH = 8, 150 mM NaCl, 1 mM MgCl₂, 50 µg/ml benzoic acid enzyme (Yeasen, 20156ES60)). The cells were lysed by three repeated freeze-thaw cycles to release AAV9 into the solution. Meanwhile, AAV9 in the cell culture medium was precipitated with 40% PEG8000 (Sigma, P2139) in 2.5 M NaCl solution, resuspended with lysis buffer, and mixed with the cell lysate. AAV in the lysate was purified in an Optiprep (Sigma, D1556-250ML) density gradient by ultracentrifugation with a 70Ti rotor (Beckman, XPN-100), followed by concentration and buffer-exchanged into a 0.001% pluronic F68/PBS (Caisson, PFL01-100ML) solution using a 100 kDa centrifugal filter tube (Millipore, UFC910096). AAV titer was measured by qPCR technology using primers AAGCTGACCCTGAAGTTCATCTGC (SEQ ID NO: 45) and CTTGTAGTTGCCGTCGTCCTTGAA (SEQ ID NO: 46) that recognize the GFP sequence.

On postnatal day 1 (P1), the mice were anesthetized with isoflurane and injected subcutaneously with an AAV9 viral vector containing the DreAM-Zf1 element (AAV-CMV-DreAM-Zf1-GFP). After 7 days, mice were intraperitoneally injected with 10 mg/kg Risdiplam. Heart, liver and skeletal muscle tissues were collected for TaqMan probe qPCR, RT-PCR and Western blot at 1, 2, 3, 4 and 5 days after administration, respectively. GFP fluorescence signals were observed under a confocal microscope (as shown in B to D in Figure4, B: the sample on the day of RIS injection was used as a control, and the changes of PSE-inclusion mRNA over time were detected by TaqMan probe method. C: RT-PCR analysis of the relative abundance of alternatively spliced mRNA with and without PSE. D-E: Western blot was performed to detect the changes in GFP protein over time using the sample on the day of RIS injection as the control for quantitative analysis. The change of GFP expression reflects *in vivo* efficacy of the Zf1 element (n=2/group).

The operation method of TaqMan-qPCR and RT-PCR is as follows: the freshly harvested tissue is rinsed in pre-cooled PBS, placed in an RNase-free EP tube, added with 1 mL of Trizol lysis solution, and ground until there are no obvious tissue blocks. After incubating at room temperature for 5 min, centrifuge the sample at 12000 g for 5 min at 4 °C, and transfer the supernatant to a new RNase-free EP tube. Add 200 µL of chloroform to the supernatant and shake vigorously to mix. Place the sample on ice for 5 min and centrifuge at 12000 g at 4°C for 15 min. The supernatant was transferred to a new EP tube, and an equal amount of anhydrous ethanol was added to the supernatant. After gently shaking to mix, the supernatant was transferred to the adsorption column of a commercially available RNA extraction kit and further eluted according to the instructions (TransZol Up Plus RNA Kit, TranGene, ER501). The extracted RNA was quantified using NanoDrop2000 (Thermofish) and then reverse transcribed using a commercially available reverse transcription reagent (HiScript^{®} III All-in-one RT SuperMix Perfect for qPCR, Vazyme, R333) according to the instructions to obtain cDNA for further TaqMan-qPCR and RT-PCR.

### TaqMan-qPCR:

1. Prepare the following mixture in a qPCR tube:

| | |
|---|---|
| 2 × AceQ qPCR Probe Master Mix | 10 µL |
| GAPDH-Primer-F (10 µM) | 0.2 µL |
| GAPDH-Primer-R (10 µM) | 0.2 µL |
| GAPDH-TaqMan Probe (10 µM) | 0.2 µL |
| Zf1-Primer-F (10 µM) | 0.2 µL |
| Zf1-Primer-R (10 µM) | 0.2 µL |
| Zf1-TaqMan Probe (10 µM) | 0.2 µL |
| 50 × ROX Reference Dye 2 | 0.4 µL |
| cDNA | 1 µL |
| ddH₂O | Up to 20 µL |

The primer sequences are as follows:

| |
|---|
| GAPDH-Primer-F (10 µM):SEQ ID NO:38 |
| GAPDH-Primer-R (10 µM):SEQ ID NO:39 |
| GAPDH-TaqMan Probe (10 µM):SEQ ID NO:40 |
| Zf1-Primer-F (10 µM):SEQ ID NO:16 |
| Zf1-Primer-R (10 µM):SEQ ID NO:41 |
| Zf1-TaqMan Probe (10 µM):SEQ ID NO:17 |

2. The qPCR reaction was performed under the following conditions:

| | | | | |
|---|---|---|---|---|
| Stage 1 | initial denaturation a | Reps: 1 | 95°C | 5 min |
| Stage 2 | cycle reaction b | Reps: 40 | 95°C | 10 sec |
| | | | 60°C | 30 sec |

3. After the reaction is completed, the amplification curve and melting curve of Real Time PCR are confirmed, and PCR quantitative calculation and statistical analysis are performed.

### RT-PCR:

1. Prepare the following mixture in a qPCR tube:

| | |
|---|---|
| 2 × Taq PCR Mix | 10 µL |
| Zf1-Primer-F1 (10 µM) | 0.2 µL |
| Zf1-Primer-R1 (10 µM) | 0.2 µL |
| cDNA | 1 µL |
| ddH₂O | Up to 20 µL |

The primer sequences are as follows:

| | |
|---|---|
| Zf1-Primer-F1 | : SEQ ID NO:18 |
| Zf1-Primer-R1 | : SEQ ID NO:41 |

2. The qPCR reaction was performed under the following conditions:

| | | | | |
|---|---|---|---|---|
| Stage 1 | initial denaturation a | Reps: 1 | 94°C | 3 min |
| | | | | |
| Stage 2 | cycle reaction b | Reps: 5 | 94°C | 30 sec |
| | | | 65°C | 30 sec |
| | | | 72°C | 15 sec |
| | | | | |
| Stage 3 | cycle reaction c | Reps: 5 | 94°C | 30 sec |
| | | | 63°C | 30 sec |
| | | | 72°C | 15 sec |
| | | | | |
| Stage 4 | cycle reaction d | Reps: 20 | 94°C | 30 sec |
| | | | 60°C | 30 sec |
| | | | 72°C | 15 sec |
| | | | | |
| Stage 5 | Full extension e | Reps: 1 | 72°C | 5 min |
| Stage 6 | Store at 12°C | | | |

3. The PCR products were separated by 1.5% agarose gel electrophoresis and visualized on a gel imaging system.

The results showed that after intraperitoneal injection of Risdiplam, pseudoexon inclusion peaked at one day after injection as detected by qPCR, and the target band was most obvious at this time point as detected by RT-PCR and gradually weakened from the second day (B to C in Figure 4). Therefore, the optimal duration of a single Risdiplam injection to enhance the effect of the DreAM-Zf1 element is 1 day.

The operation method of Western Blot is as follows: after washing the fresh tissue with cold PBS, the tissue is fully disrupted using a homogenizer in RIPA buffer (25mM Tris PH7.0-8.0, 150mM NaCl, 0.1% SDS, 0.5% sodium deoxycholate, 1% Triton X-100, protease inhibitor (Solarbio, A8260)). After lysis on ice for 10 minutes, the lysate was centrifuged at 12,000 rpm for 15 minutes at 4°C, and the supernatant was collected, and the protein concentration was determined using the BCA assay (TransGen, DQ111-01).

After determining the protein concentration of the samples, the protein concentrations in the lysates were adjusted to the same level using RIPA buffer and diluted in 4× SDS sample buffer (Solarbio, P1016). After the dilution solution was boiled for 10 minutes, a 4% to 15% gradient gel (TransGen, DG101-01) was prepared, 20 µg of protein from each sample was loaded onto the gel and then transferred to a PVDF membrane. After incubation in TBST containing 5% skim milk at room temperature for one hour, the membranes were incubated with primary antibodies (Ms-Anti-GAPDH, 1:10000, TransGen, HC301; Ms-Anti-GFP, 1:5000, TransGen, HT801) at 4°C overnight. The next day, the membranes were washed three times with TBST for five minutes each timeand then incubated with secondary antibody (HRP-Gt-Anti-Ms, 1:5000, BBI, D110087-0100) for 1 hour. After washing three times with TBST for five minutes each time, the membrane was evenly incubated with ECL substrate (Solarbio, PE0010) and imaged using the iBright CL1500 imaging system (Thermo Fisher Scientific).

Western blot results showed that in the presence of Risdiplam, the DreAM-Zf1 element could enhance the expression of the reporter gene GFP in heart, liver, and skeletal muscle tissues, and the specificity in heart and skeletal muscle was better than that in liver tissue (D to E in Figure 4).

The mouse heart, liver, and skeletal muscle tissues were embedded in OCT, sectioned frozen the tissue, cell membranes were labeled with WGA, and photos were taken under a confocal microscope to examine the regulation of GFP fluorescent protein expression by the DreAM-Zf1 element before and after Risdiplam injection. The results showed that after the injection of Risdiplam, the expression signals of the reporter gene GFP in the heart, liver, and skeletal muscle tissues were significantly enhanced (F in Figure 4).

Furthermore, the inventors administered the mice, which were pre-injected with AAV-CMV-DreAM-Zf1-GFP, with different doses (0, 2, 10, 50 mg/kg) of Risdiplam, and detected the dynamic changes of CMV-DreAM-Zf1 activity by TaqMan-qPCR, RT-PCR and Western blot. The results showed that the extent of pseudoexon inclusion and the GFP protein level in mice were significantly enhanced after Risdiplam administration and were positively correlated with the dosage.

The results of this example show that the DreAM-Zf1 element of the present disclosure can control the expression of the target gene by administering Risdiplam, and this regulation method is time- and dose-dependent.

### Example 5 Comparison of CMV-DreAM-Zf1 and X^{on} elements

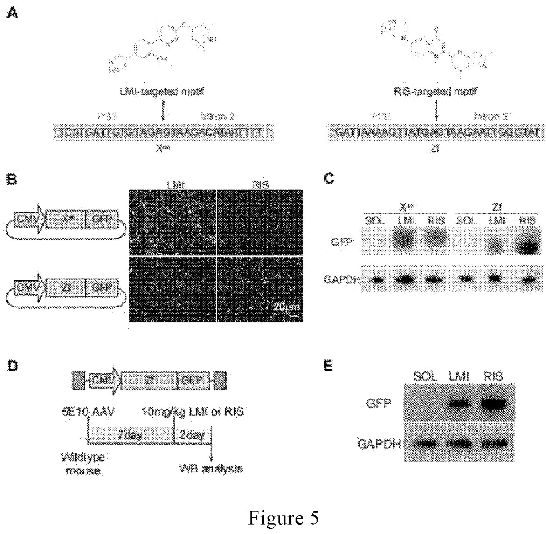

The published X^{on} element and the CMV-DreAM-Zf1 element of the present disclosure have similar drug recognition motifs (A in Figure 5), and with only a single base difference between two elements. Therefore, the inventors constructed two plasmids: CMV-X^{on}-GFP (the construction methods were followed as described by Monteys, A.M., et al., Regulated control of gene therapies by drug-induced splicing. Nature 596, 291-295 (2021)) and CMV-DreAM-Zf1-GFP, two plasmids were used to transfect 293T cells, and 1 µM of LMI070 and Risdiplam were used to regulate and induce the expression of the target gene GFP, respectively. Western blot experimental analysis and fluorescence microscopy were used to observe the GFP signal. The results showed that in both CMV-DreAM-Zf1 and X^{on} system, compared with the control group (Sol) without medication, the expression of GFP was significantly increased after administration of LMI070 and Risdiplam (B to C in Figure 5, B is the comparison of GFP fluorescence signals, and C is the comparison result of Western blot). This suggests that both systems can be modulated using both LMI070 and Risdiplam.

In addition, newborn mice (P1) were injected with AAV-CMV-DreAM-Zf1-GFP, and 7 days later, 10 mg/kg Risdiplam and LMI070 were intraperitoneally injected into the mice, respectively. 2 days later, cardiac tissues were harvested for Western blot analysis (Figure 5, D and E). The results showed that the administration of Risdiplam and LMI070 could significantly enhance the expression of GFP in the mouse cardiac tissue. This suggests that the DreAM-Zf1 system is regulated by both LMI070 and Risdiplam in vivo.

The results of this example show that the RNA splicing modulator to which the DreAM-Zf1 element of the present disclosure can respond can be selected from either of the two drugs, LMI070 and Risdiplam.

### Example 6 DreAM-Zf1 element regulates the activation of the Yap gene

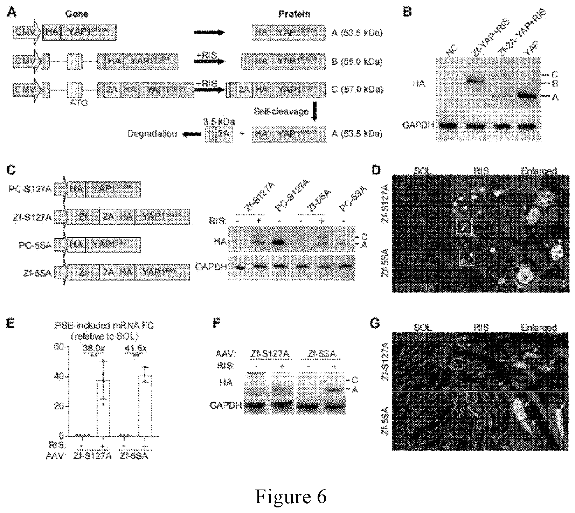

Yap gene is an important gene regulating cardiomyocyte proliferation and myocardial regeneration. YAP activation is important for the treatment of heart diseases, such as myocardial infarction that involves cardiomyocyte death. To verify that the DreAM-Zf1 element can regulate downstream genes used for gene therapy, such as the Yap gene, the inventors constructed a CMV-DreAM-Zf1-HA-Yap1^{S127A} plasmid. In this plasmid, Yap1^{S127A} refers to a YAP mutant with an S>A mutation at amino acid position 127 (SEQ ID NO:42). HA was used as a tag protein to detect YAP protein whose expression was controlled by Zf1. In order to prevent DreAM-Zf1 from introducing extraneous amino acids with unknown function to the N-terminus of the YAP1 protein, the inventors also constructed the plasmid CMV-DreAM-Zf1-2A-HA-Yap1^{S127A}. In this plasmid, the inventors inserted a short self-cleaved peptide P2A between the amino acids encoded by DreAM-Zf1 and the amino acids of YAP1, thereby seamlessly expressing the complete Yap1^{S127A} protein. The specific working principle and process are shown in A of Figure 6. As a positive control, the inventors constructed CMV-HA-Yap1^{S127A} plasmid.

The method for plasmid construction is as follows: the DreAM-Zf1 sequence is derived from the vector plasmid constructed in Example 3, and the Yap1^{S127A} DNA fragment is entrusted to Suzhou GENEWIZ Biotechnology Co., Ltd. (Suzhou, Jiangsu, China) for gene synthesis. CMV promoter and HA tag were derived from plasmid sequences published by Addgene. DNA segment assembly and plasmid construction were performed by seamless cloning reactions.

After the above three plasmids were transfected into 293T cells, the medium was changed and drugs were added after 6 hours, and the cells were collected for Western blot analysis after 24 hours. The CMV-DreAM-Zf1-HA-Yap1^{S127A} and CMV-DreAM-Zf1-2A-HA-Yap1^{S127A} groups were treated with 1 µM Risdiplam, and the negative control group was the group without plasmid transfection (NC). As shown in B in Figure 6, band A corresponds to the HA-YAP, band B corresponds to the DreAM-Zf1-HA-YAP, and band C corresponds to the DreAM-Zf1-2A-HA-YAP in which 2A did not undergo self-cleavage. These results suggest that the P2A short peptide can undergo self-cleavage to remove the amino acids encoded by DreAM-Zf1, thereby seamlessly expressing the complete Yap1^{S127A} protein.

YAP1^{5SA} (referring to a YAP mutant with five serine-to-alanine substitutions, SEQ ID NO: 43) has less phosphorylation modification and a stronger ability to promote myocardial proliferation than Yap1^{S127A}. The inventors then constructed CMV-DreAM-Zf1-2A-HA-YAP1^{5SA} plasmid and CMV-HA-YAP1^{5SA} plasmid (positive control). The two plasmids were transfected into 293T cells respectively, and the medium was changed after 6 hours and 1 µM Risdiplam or SOL control treatment was performed. The negative control group was the group without plasmid transfection (NC). After 24 h of treatment, cells were collected for Western blot and immunofluorescence staining, and HA-positive cells were observed under confocal microscopy.

The results showed that after treatment with Risdiplam, cells transfected with DreAM-Zf1-2A-HA-YAP expressed two proteins: HA-YAP protein before 2A self-cleavage (band C in Figure 6C) and HA-YAP protein after self-cleavage (band A in Figure 6C). Confocal microscopy imaging showed that Yap1^{S127A} and YAP1^{5SA} were expressed in 293T cells after Risdiplam treatment, and the YAP protein had obvious nuclear localization (D in Figure 6, where WGA marked the nucleus, HA marked the Zf-controlled expressed YAP protein, and the high-magnification image confirmed the nuclear localization of the YAP mutant). These results indicate that the DreAM-Zf1 element can be induced by Risdiplam in vitro and regulate the expression of multiple downstream target genes.

To further verify whether DreAM-Zf1 is effective in regulating YAP gene expression in vivo, the inventors constructed the vectors by tandemly linking the cardiomyocyte-specific Tnnt2 promoter, DreAM-Zf1 element, and Yap1^{S127A}or YAP1^{5SA} reporter genes, and then packaged these vectors into a new AAV9 vector for in vivo studies in mice.

The method for constructing AAV plasmid was as follows: The DreAM-Zf1 sequence DNA fragment was commissioned to Suzhou GENEWIZ Biotechnology Co., Ltd. (Suzhou, Jiangsu, China) for gene synthesis. cTnnt2/hTnnt2, Yap1^{S127A} and YAP1^{5SA} genes were derived from publicly available plasmid sequences on Addgene. DNA segment assembly and plasmid construction were performed by seamless cloning reactions.

The inventors packaged the above plasmid into AAV. Newborn mice (P1) were injected with AAV, and 7 days later, Risdiplam (10 mg/kg) was injected intraperitoneally. One day later, the mouse heart tissues were collected for TaqMan-qPCR, Western blot, and immunofluorescence staining confocal imaging analysis. The results of TaqMan-qPCR showed that at the mRNA splicing level, Risdiplam increased the pseudoexon inclusion of Zf1 element by about 40-fold in cardiac tissue (Figure 6E). Western blot results showed that at the protein level, Risdiplam significantly increased the expression of Yap1^{S127A} and YAP1^{5SA} in in cardiac tissue (F in Figure 6).Only the band after 2A self-cleavage was detectable by Western blot (band A in Figure 6), but the band before self-cleavage is undetectable (band C in Figure 6), indicating that the self-cleavage peptide has a high efficiency in vivo. Immunofluorescence staining results showed that Risdiplam significantly increased the expression of Yap1^{S127A} and YAP1^{5SA} in cardiomyocytes and YAP was localized in the nucleus (G in Figure 6).

### Example 7 Application of the DreAM-Zf1 element-regulated gene editing system

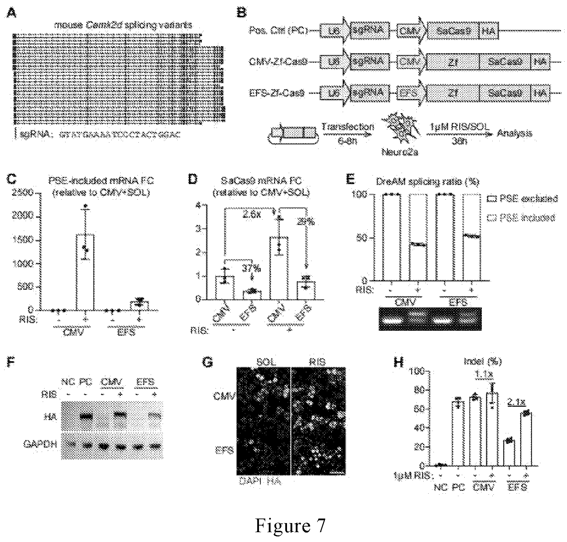

In this example, the inventors investigated whether the DreAM-Zf1 element could regulate Cas9-mediated gene editing. To this end, the inventors tandemly linked the CMV or EFS promoter, the DreAM-Zf1 element, and SaCas9 to construct a plasmid for gene editing, which was then used in Neuro2a cells for verification. Both EFS and CMV are promoters that can activate systemic transcription. The activity of EFS is weaker than that of CMV. The above plasmid also comprises the expression element of sgRNA (SEQ ID NO: 44, A in Figure 7) targeting the Camk2d gene. Camk2d, the gene encoding CaMKIIδ, which is a key therapeutic target for heart disease.

The method for plasmid construction is as follows: DreAM-Zf1 and sgRNA sequence DNA fragments were commissioned to Suzhou GENEWIZ Biotechnology Co., Ltd. (Suzhou, Jiangsu, China) for gene synthesis. CMV, EFS, SaCas9, and U6 are derived from plasmids publicly released by Addgene. DNA segment assembly and plasmid construction were performed by seamless cloning reactions. Three plasmids: U6-sgRNA-CMV-SaCas9-HA, U6-sgRNA-CMV-DreAM-Zf1-SaCas9-HA and U6-sgRNA-EFS-DreAM-Zf1-SaCas9-HA were constructed.

Neuro2a cells were transfected with the above plasmids, and the medium was changed after 6 h. The cells were treated with Risdiplam (1 µM) or solvent for 36 h, and the cells were collected for TaqMan-qPCR, RT-PCR, Western blot, immunofluorescence and amplicon sequencing analysis (B in Figure 7). qPCR results showed that the level of pseudoexon inclusion in mRNA induced by Risdiplam was significantly higher under the CMV promoter than under the EFS promoter, reaching up to 2000-fold (Figure 7C). The expression level of SaCas9 mRNA induced by Risdiplam increased by about 2.6-fold. The ability of EFS promoter to activate SaCas9 transcription was significantly lower than that of CMV promoter, which was about 30% of the transcription activation of CMV promoter, whether or not the drug was administered (D in Figure 7), which verified the fact that CMV promoter activity was higher than that of EFS.

RT-PCR analysis showed that after Risdiplam treatment, the DreAM-Zf1 elements activated by two different promoters had significantly increased levels of pseudoexon inclusion in mRNA (Figure 7E). Western blot and immunofluorescence staining results showed that after Risdiplam induction, the protein expression level of SaCas9 was also significantly increased in the DreAM-Zf1 elements activated by two different promoters: CMV and EFS (F and G in Figure 7). The results of amplicon sequencing showed that the incidence of base insertion/deletion mutations (indels) in the DreAM-Zf1 element activated by the CMV promoter did not increase significantly after Risdiplam treatment, while the incidence of base insertion/deletion mutations (indels) in the DreAM-Zf1 element activated by the EFS promoter increased by 2.1-fold after Risdiplam treatment. When not treated with Risdiplam, the incidence of indels produced by the SaCas9 gene editing system induced by EFS promoter-activated DreAM-Zf1 element was significantly lower than that in the CMV promoter group (H in Figure 7).

The above results show that the DreAM-Zf1 element is suitable for application scenarios of controlling gene editing systems. However, since gene editing is a highly sensitive system that is sensitive to low-expression Cas9, the DreAM-Zf1 element needs to be combined with a promoter with weaker transcriptional activation ability to achieve control of gene editing.

### Example 8 Further truncation of the DreAM-Zf1 element

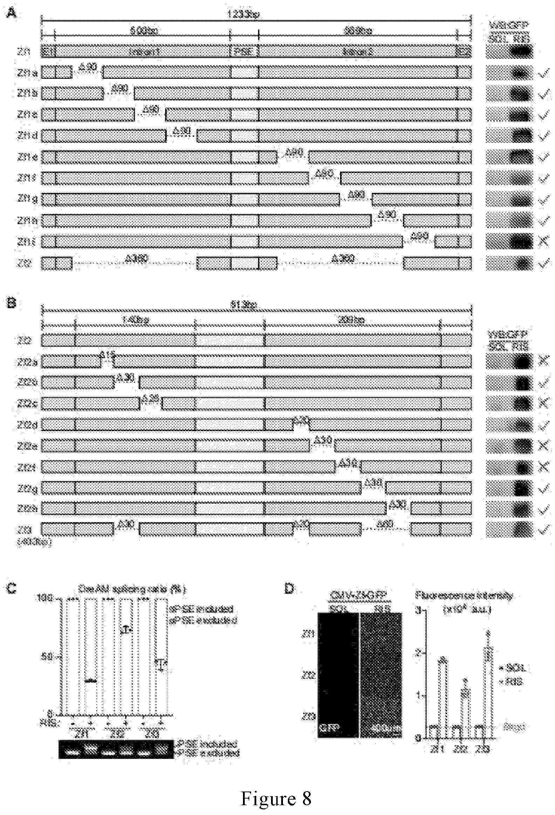

The method for truncating the DreAM-Zf1 element is as follows:
The inventors believe that the (pseudo) exon of the DreAM-Zf1 element and its adjacent intronic fragment are essential for the function of DreAM, but there may be non-essential nucleic acid fragments in the middle of the two introns. Therefore, the inventors fragmented the middle segments of introns 1 and 2 of the DreAM-Zf1 element into several small fragments, deleted them separately and tested their impact on the function of DreAM. Based on the existing DreAM-Zf1 element, primers were designed to amplify multiple fragments of the vector plasmid containing the DreAM-Zf1 element and perform seamless cloning to construct comprising truncated element vectors (Zf1a-i) (A in Figure 8).

Subsequently, the inventors transfected HEK293T cells with plasmids comprising the truncated elements, and after 24 hours of Risdiplam treatment, the difference in GFP protein expression caused by Risidiplam was detected via Western Blot (A in Figure 8). The cells transfected with CMV-GFP plasmid were used as the positive control group, and the cells transfected with CMV-(ATG)GFP plasmid were used as the negative control group.

The data showed that, except for Zf1i, the other truncated fragments of the Zf1a-i element had no significant impact on the function of DreAM. The inventors deleted these small fragments that did not affect the characteristics of the DreAM-Zf1 element and obtained the DreAM-Zf2 element (A in Figure 8).

Based on the DreAM-Zf2 element, the inventors further truncated the elements by deleting small fragments. Similarly, primers were designed to amplify multiple fragments of the vector comprising the DreAM-Zf2 element and perform seamless cloning to construct vectors containing truncated elements. HEK293T cells were transfected with plasmids containing truncated elements, respectively. The cells transfected with CMV-GFP plasmid were used as the positive control group, and the cells transfected with CMV-(ATG)GFP plasmid were used as the negative control group. After 24 h of Risdiplam treatment, the difference in GFP protein expression caused by Risdiplam was detected by Western Blot (B in Figure 8).

The data shows that deletion of several small fragments within the DreAM-Zf2 element does not have a significant impact on the function of DreAM. The inventors deleted these small fragments that did not affect the characteristics of the DreAM-Zf2 element and obtained the DreAM-Zf3 element (B in Figure 8).

HEK293T cells were transfected with plasmids containing DreAM-Zf1, DreAM-Zf2, or DreAM-Zf3 elements, respectively. The cells transfected with CMV-GFP plasmid were used as the positive control group, and the cells transfected with CMV-(ATG)GFP plasmid were used as the negative control group. After 24 hours of Risdiplam treatment, the fluorescence signal intensity was observed and quantitatively analyzed. At the same time, the cells were collected and the pseudoexons inclusion regulated by Risidiplam-induced nucleic acid modulatory elements were quantitatively detected by RT-PCR assay (C and D in Figure 8). The data showed that the truncated DreAM-Zf2 and DreAM-Zf3 elements still have similar properties to the DreAM-Zf1 element, while being significantly shortened in length.

The nucleotide sequence of the DreAM-Zf2 element is:

ACTGACCAACAGCGTGAAGACATTACATCCAGTGCACAGGTAATTGAATTCTT
GTTGGATTACTGTCTCTAAGGCCTGGGAGTTAGTAGATATGTGTGTGGGTTAAATGA
CTTAAGCATACATCAAAAGACTGAGAAAGGCTCTTAACATTAAAAGATGGGCCttttttt
ttCTTTTAGGTTAAAGGATTACATATTAAAAATATTGTATAGACTCTTTAAGGCCAAA
GAAGTGTGCTCATTTCTCCCGATTAAAAGTTATGAGTAAGAATTGGGTATTTTGCCT
TTTGGGGGTGGGAGAtttgaaacagggtttcCAGTGAGATAGCTCTGCAGGTAAAAGGGAGA
CTGACCCCCAACTTGTACTCTGGCCGTGGCAGTCATGTGCACACAAGTAAACTGAA
TAAATATAAAGAGCAGATTTTAGAGTGTTTTGGCTTTAAATTAATTCTATATTCTAA
CTCTATTTGCAGTTTGCATTGAGACTCCTTGCATTCCGTCAGATACACAAAGTT(Zf2,
SEQ ID NO:11).

The nucleotide sequence of the first intron of the DreAM-Zf2 element is:

GTAATTGAATTCTTGTTGGATTACTGTCTCTAAGGCCTGGGAGTTAGTAGATAT
GTGTGTGGGTTAAATGACTTAAGCATACATCAAAAGACTGAGAAAGGCTCTTAACA
TTAAAAGATGGGCCtttttttttCTTTTAG(SEQ ID NO:4).

The nucleotide sequence of the second intron of the DreAM-Zf2 element is:

GTAAGAATTGGGTATTTTGCCTTTTGGGGGTGGGAGAtttgaaacagggtttcCAGTGAG
ATAGCTCTGCAGGTAAAAGGGAGACTGACCCCCAACTTGTACTCTGGCCGTGGCAG
TCATGTGCACACAAGTAAACTGAATAAATATAAAGAGCAGATTTTAGAGTGTTTTG
GCTTTAAATTAATTCTATATTCTAACTCTATTTGCAG(SEQ ID NO:7).

The nucleotide sequence of the DreAM-Zf3 element is:

ACTGACCAACAGCGTGAAGACATTACATCCAGTGCACAGGTAATTGAATTCTT

GTTGGATTACTGTCTCTAAGGCCTGGGAGTTAGCATACATCAAAAGACTGAGAAAG

GCTCTTAACATTAAAAGATGGGCCtttttttttCTTTTAGGTTAAAGGATTACATATTAAAA

ATATTGTATAGACTCTTTAAGGCCAAAGAAGTGTGCTCATTTCTCCCGATTAAAAGT

TATGAGTAAGAATTGGGTATTTTGCCTTTTGGGGGTGGGAGTGAGATAGCTCTGCA

GGTAAAAGGGAGACTGACCCCCAACTTGTACTCTGGCCGTGGCTTTAAATTAATTCT

ATATTCTAACTCTATTTGCAGTTTGCATTGAGACTCCTTGCATTCCGTCAGATACAC

AAAGTT(Zf3, SEQ ID NO:12).

The nucleotide sequence of the first intron of the DreAM-Zf3 element is:

GTAATTGAATTCTTGTTGGATTACTGTCTCTAAGGCCTGGGAGTTAGCATACAT

CAAAAGACTGAGAAAGGCTCTTAACATTAAAAGATGGGCCtttttttttCTTTTAG(SEQ ID

NO:5).

The nucleotide sequence of the second intron of the DreAM-Zf3 element is:

GTAAGAATTGGGTATTTTGCCTTTTGGGGGTGGGAGTGAGATAGCTCTGCAGG
TAAAAGGGAGACTGACCCCCAACTTGTACTCTGGCCGTGGCTTTAAATTAATTCTAT
ATTCTAACTCTATTTGCAG(SEQ ID NO:8).

Finally, it should be noted that the above embodiments are only used to illustrate the technical solutions of the present disclosure, rather than to limit them. Although the present disclosure has been described in detail with reference to the aforementioned embodiments, those skilled in art should understand that they can still modify the technical solutions described in the aforementioned embodiments, or replace some, or all of the technical features therein by equivalents. However, these modifications or replacements do not cause the essence of the corresponding technical solutions to deviate from the scope of the technical solutions of the embodiments of the present disclosure.

## Claims

1. An alternative splicing modulatory element, which comprises a first exon, a first intron, a pseudoexon, a second intron and a second exon in sequence from the 5' end to the 3' end; the pseudoexon comprises a nucleotide sequence having at least 95% identity to the sequence as shown in SEQ ID NO:1; the junction between the pseudoexon and the second intron comprises a binding site for a small-molecule alternative splicing modifier drug; and the alternative splicing modulatory element comprises a sole start codon exclusively in the pseudoexon.

2. The alternative splicing modulatory element according to claim 1, wherein the binding site for a small-molecule alternative splicing modifier drug comprises the nucleotide sequence ATGAGTA, wherein ATGA belongs to a pseudoexon and GTA belongs to a second intron; or
the binding site for a small-molecule alternative splicing modifier drug comprises the nucleotide sequence AGAGTA, wherein AGA belongs to the pseudoexon and GTA belongs to the second intron.

3. The alternative splicing modulatory element according to claim 1 or 2, wherein the sequence of the first exon immediately followed by the sequence of the first intron is CAG, and the sequence of the first intron immediately following the sequence of the first exon is GTA; and/or,
the sequence of the first intron immediately followed by the sequence of the pseudoexon is TAG, and the sequence of the pseudoexon immediately following the sequence of the first intron is GTT; and/or,
the sequence of the second intron immediately followed by the sequence of the second exon is CAG, and the sequence of the second exon immediately following the sequence of the second intron is TTT.

4. The alternative splicing modulatory element according to any one of claims 1 to 3, wherein the first intron comprises a nucleotide sequence having at least 95% identity to the sequences as shown in SEQ ID NO: 19 and 20; and the second intron comprises a nucleotide sequence having at least 95% identity to the sequence as shown in SEQ ID NO: 21.

5. The alternative splicing modulatory element according to any one of claims 1 to 4, wherein the nucleotide length of the first intron is 41 to 500 bp, preferably 41 to 410 bp, preferably 41 to 140 bp, preferably 41 to 110 bp; the nucleotide length of the second intron is 60 to 569 bp, preferably 60 to 479 bp, preferably 60 to 209 bp, preferably 60 to 129 bp.

6. The alternative splicing modulatory element according to any one of claims 1 to 5, wherein the first intron comprises a nucleotide sequence having at least 95% identity to the sequence as shown in any one of SEQ ID NOs: 3 to 5, 22 to 25; and the second intron comprises a nucleotide sequence having at least 95% identity to the sequence as shown in any one of SEQ ID NOs: 6 to 8, 26 to 32.

7. The alternative splicing modulatory element according to any one of claims 1 to 6, wherein
(a) the first intron comprises a nucleotide sequence having at least 95% identity to the sequence as shown in SEQ ID NO:5; the second intron comprises a nucleotide sequence having at least 95% identity to the sequence as shown in SEQ ID NO:8; or,
(b) the first intron comprises a nucleotide sequence having at least 95% identity to the sequence as shown in SEQ ID NO:4; the second intron comprises a nucleotide sequence having at least 95% identity to the sequence as shown in SEQ ID NO:7; or,
(c) the first intron comprises a nucleotide sequence having at least 95% identity to the sequence as shown in SEQ ID NO:3; and the second intron comprises a nucleotide sequence having at least 95% identity to the sequence as shown in SEQ ID NO:6.

8. The alternative splicing modulatory element according to any one of claims 1 to 7, wherein the first exon comprises a nucleotide sequence having at least 95% identity to the sequence as shown in SEQ ID NO: 2; and/or the second exon comprises a nucleotide sequence having at least 95% identity to the sequence as shown in SEQ ID NO: 9.

9. The alternative splicing modulatory element according to any one of claims 1 to 8, wherein the alternative splicing modulatory element comprises a nucleotide sequence having at least 95% identity to the sequence as shown in any one of SEQ ID NOs: 10 to 12.

10. A nucleic acid molecule comprising the alternative splicing modulatory element according to any one of claims 1 to 9 and a target gene positioned at the 3' end of the alternative splicing modulatory element.

11. The nucleic acid molecule according to claim 10, wherein no nucleic acid sequence exists between the 3' end of the alternative splicing modulatory element and the 5' end of the target gene.

12. The nucleic acid molecule according to claim 10, wherein a sequence encoding a protease cleavage site exists between the 3' end of the alternative splicing modulatory element and the 5' end of the target gene, wherein the protease cleavage site is cleaved by a mammalian protease or is self-cleaved, and the number of bases in the sequence encoding the protease cleavage site is an integer multiple of 3.

13. The nucleic acid molecule according to any one of claims 10 to 12, wherein the 5' end of the alternative splicing modulatory element is operably linked to a promoter sequence.

14. The nucleic acid molecule according to claim 13, wherein the promoter sequence is a mammalian cell constitutive promoter, a mammalian cell specific promoter, a mammalian non-coding RNA promoter or a prokaryotic cell promoter.

15. The nucleic acid molecule according to claim 14, wherein the mammalian cell constitutive promoter is CMV, CAG, CBG, EF1a, PGK1 or Ubc; the mammalian cell specific promoter is Tnnt2, Nppa, Myl2, Mck, Nkx2.5, Syn, Mecp2, TBG, Pdx1, K14, Rpe65 or SP-C; the mammalian non-coding RNA promoter is U6 or H1; the prokaryotic cell promoter is T7, T3 or SP6.

16. The nucleic acid molecule according to any one of claims 10 to 15, wherein the nucleic acid molecule further comprises a post-transcriptional modulatory element and/or a target sequence of a microRNA.

17. The nucleic acid molecule of claim 16, wherein the post-transcriptional modulatory element (PRE) comprises a post-transcriptional modulatory element derived from hepatitis B (HPRE), bat (BPRE), ground squirrel (GSPRE), Arctic squirrel (ASPRE), duck (DPRE), chimpanzee (CPRE), woolly monkey (WMPRE) or woodchuck (WPRE);
the microRNA is selected from miR122, miR199, miR7, miR148, miR1 or miR208;
optionally, the post-transcriptional modulatory element and/or the target sequence of the microRNA is disposed at the 3' end of the target gene.

18. The nucleic acid molecule according to any one of claims 10 to 17, wherein the nucleic acid molecule further comprises a polyadenylation signal (polyA), optionally wherein the polyA is positioned at the 3' end of the target gene.

19. The nucleic acid molecule according to claim 18, wherein the polyA signal is SV40 polyA, human growth hormone (HGH) polyA, bovine growth hormone (BGH) polyA, β-globin polyA, α-globin polyA, ovalbumin polyA, κ-light chain polyA or synthetic polyA.

20. The nucleic acid molecule according to any one of claims 10 to 19, wherein the alternative splicing modulatory element can bind to a small-molecule alternative splicing modifier drug, and the small-molecule alternative splicing modifier drug is LMI070, a derivative having the same binding site as LMI070 on the alternative splicing modulatory element, or a pharmaceutically acceptable salt of LMI070; or the small-molecule alternative splicing modifier drug is Risdiplam, a derivative having the same binding site as Risdiplam on the alternative splicing modulatory element, or a pharmaceutically acceptable salt of Risdiplam.

21. The nucleic acid molecule according to any one of claims 10 to 20, wherein the gene encoding the protein is a gene encoding a nuclease, and/or a gene encoding a therapeutic protein, and/or a gene encoding a reporter or marker protein, and/or a gene encoding a non-coding RNA;
preferably, the nuclease includes a transcription activator-like effector nuclease (TALEN), a zinc finger nuclease (ZFN), a CRISPR associated protein (Cas protein) or a derivative with the same function thereof, and further preferably, the Cas protein comprises a Cas9 protein, a Cas12 protein or a Cas13 protein;
preferably, the therapeutic protein includes YAP, LMNA, RPE65, SMN1, FVIII, FIX or their functionally equivalent derivatives;
preferably, the marker protein includes eGFP, tdTomato, mCherry, Luciferase, SEAP, CAT, GST, β-GUS, β-Gal or their functionally equivalent derivatives;
preferably, the non-coding RNA includes at least one of miRNA, shRNA or LncRNA having gene inhibition function.

22. A construction method of the nucleic acid molecule according to any one of claims 10 to 21, which comprises connecting the target gene with the 5'-end start codon removed to the 3' end of the alternative splicing modulatory element according to any one of claims 1 to 9, so that the target gene and the start codon in the alternative splicing modulatory element use the same reading frame.

23. A transcript, in the presence of a small-molecule alternative splicing modifier drug, which is the transcript of the nucleic acid molecule according to any one of claims 10 to 21 or the transcript of the nucleic acid molecule obtained by the construction method according to claim 22; or in the absence of a small-molecule alternative splicing modifier drug, which is the transcript of the nucleic acid molecule according to any one of claims 10 to 21 or the transcript of the nucleic acid molecule obtained by the construction method according to claim 22;
the small-molecule alternative splicing modifier drug is LMI070, a derivative having the same binding site as LMI070 on the alternative splicing modulatory element, or a pharmaceutically acceptable salt of LMI070; or, the small-molecule alternative splicing modifier drug is Risdiplam, a derivative having the same binding site as Risdiplam on the alternative splicing modulatory element, or a pharmaceutically acceptable salt of Risdiplam.

24. A vector comprising the alternative splicing modulatory element according to any one of claims 1 to 9, the nucleic acid molecule according to any one of claims 10 to 21, the nucleic acid molecule obtained by the construction method according to claim 22, or the transcript according to claim 23.

25. The vector according to claim 24, wherein the vector is a DNA or RNA vector, or the vector is double-stranded or single-stranded.

26. The vector according to claim 24, wherein the vector is a viral vector.

27. The vector of claim 26, wherein the viral vector is an adeno-associated virus (AAV) vector, a chimeric AAV vector, an adenoviral vector, a retroviral vector, a lentiviral vector, a herpes simplex virus vector, a baculoviral vector, or any mutant or derivative thereof.

28. A recombinant virus comprising the alternative splicing modulatory element according to any one of claims 1 to 9, the nucleic acid molecule according to any one of claims 10 to 21, the nucleic acid molecule obtained by the construction method according to claim 22, the transcript according to claim 23, or the vector according to any one of claims 24 to 27.

29. The recombinant virus of claim 28, wherein the recombinant virus is an adeno-associated virus (AAV), a chimeric AAV, an adenovirus, a retrovirus, a lentivirus, a herpes simplex virus, a baculovirus, or any mutant or derivative thereof.

30. The recombinant virus according to claim 29, wherein the adeno-associated virus is one or more of the following: AAV1, AAV2, AAV3, AAV4, AAV5, AAV6, AAV7, AAV8, AAV9, AAV10, AAV11, AAV12, AAVrh8, AAVrh10, AAVrh36, AAVrh37, AAVrh74, AAVrh79, AAV-DJ, AAV-DJ/8, AAV.Anc80, AAV.Anc80L65, AAV-PHP.B, AAV-PHP.B2, AAV-PHP.B3, AAV-PHP.A, AAV-PHP.eB, AAV-PHP.S, AAV2i8, MyoAAV, AAVMYO, AAV.CPP.16 capsid serotype or its variants.

31. A cell comprising one or more alternative splicing modulatory elements according to any one of claims 1 to 9, one or more nucleic acid molecules according to any one of claims 10 to 21, the nucleic acid molecule obtained by the construction method according to claim 22, the transcript according to claim 23, one or more vectors according to any one of claims 24 to 27, or one or more recombinant viruses according to any one of claims 28 to 30.

32. The cell according to claim 31, wherein the cell is a human cell;
preferably, the human cells are cells isolated from the body;
preferably, the human cells are immortalized cell lines or cancer cells.

33. The cell according to claim 32, wherein the human cell is a cardiac cell, a muscle cell, a neuron, a hepatocyte cell, a splenocyte cell, a pulmonary cell or a renal cell;

34. A pharmaceutical composition, comprising one or more alternative splicing modulatory elements according to any one of claims 1 to 9, one or more nucleic acid molecules according to any one of claims 10 to 21, the nucleic acid molecule obtained by the construction method according to claim 22, the transcript according to claim 23, one or more vectors according to any one of claims 24 to 27, or one or more recombinant viruses according to any one of claims 28 to 30, or one or more cells according to any one of claims 31 to 33.

35. A method for modulating the expression of a target gene based on a small-molecule alternative splicing modifier drug, comprising: contacting one or more nucleic acid molecules according to any one of claims 10 to 21, the nucleic acid molecule obtained by the construction method according to claim 22, the transcript according to claim 23, one or more vectors according to any one of claims 24 to 27, one or more recombinant viruses according to any one of claims 28 to 30, one or more cells according to any one of claims 31 to 33, and one or more pharmaceutical compositions according to claim 34 with one or more small-molecule alternative splicing modifier drugs;
wherein, the small-molecule alternative splicing modifier drug is LMI070, a derivative having the same binding site as LMI070 on the alternative splicing modulatory element, or a pharmaceutically acceptable salt of LMI070; or, the small-molecule alternative splicing modifier drug is Risdiplam, a derivative having the same binding site as Risdiplam on the alternative splicing modulatory element, or a pharmaceutically acceptable salt of Risdiplam.

36. A kit, comprising:
(1) one or more nucleic acid molecules according to any one of claims 10 to 21, nucleic acid molecules obtained by the construction method according to claim 22, transcripts according to claim 23, one or more vectors according to any one of claims 24 to 27, one or more recombinant viruses according to any one of claims 28 to 30, one or more cells according to any one of claims 31 to 33, one or more pharmaceutical compositions according to claim 34; and/or,
(2) one or more small-molecule alternative splicing modifier drugs; wherein the small-molecule alternative splicing modifier drug is LMI070, a derivative having the same binding site as LMI070 on the alternative splicing modulatory element, or a pharmaceutically acceptable salt of LMI070; or, the small-molecule alternative splicing modifier drug is Risdiplam, a derivative having the same binding site as Risdiplam on the alternative splicing modulatory element, or a pharmaceutically acceptable salt of Risdiplam.

37. (a) and (b) for use in modulating the expression of a target gene or in preparation of a gene therapy drug;
wherein (a) is selected from one or more alternative splicing modulatory elements according to any one of claims 1 to 9, one or more nucleic acid molecules according to any one of claims 10 to 21, nucleic acid molecules obtained by the construction method according to claim 22, transcripts according to claim 23, one or more vectors according to any one of claims 24 to 27, one or more recombinant viruses according to any one of claims 28 to 30, one or more cells according to any one of claims 31 to 33, one or more pharmaceutical compositions according to claim 34, or the kit according to claim 36;
(b) is a small-molecule alternative splicing modifier drug; wherein the small-molecule alternative splicing modifier drug is LMI070, a derivative having the same binding site as LMI070 on the alternative splicing modulatory element, or a pharmaceutically acceptable salt of LMI070, or the small-molecule alternative splicing modifier drug is Risdiplam, a derivative having the same binding site as Risdiplam on the alternative splicing modulatory element, or a pharmaceutically acceptable salt of Risdiplam;
preferably, the indications that the gene therapy drug can treat include ischemic cardiac disease, cardiomyopathy, muscular dystrophy, neuromuscular disease, progeria, retinitis pigmentosa, congenital amaurosis, age-related macular degeneration, spinal muscular atrophy or hemophilia.

38. A probe targeting the junction between a pseudoexon and a second exon in the transcript of claim 23 in the presence of a small-molecule alternative splicing modifier drug.

39. The probe according to claim 38, wherein the probe comprises a nucleotide sequence having at least 95% identity to the nucleotide sequence of SEQ ID NO:16.

40. A method for detecting the alternative splicing modulatory element according to any one of claims 1 to 9, one or more nucleic acid molecules according to any one of claims 10 to 21, the nucleic acid molecule obtained by the construction method according to claim 22, the transcript according to claim 23, one or more vectors according to any one of claims 24 to 27, one or more recombinant viruses according to any one of claims 28 to 30, one or more cells according to any one of claims 31 to 33, and one or more pharmaceutical compositions according to claim 34, wherein the method comprises a qPCR amplification step, and the probe according to claim 38 or 39 is used in the qPCR amplification step.

41. The method according to claim 40, wherein the forward primer used in the qPCR amplification step is selected from SEQ ID NO: 17.

42. The method according to claim 40, the method further comprises an RT-PCR amplification step before the qPCR amplification step, and preferably, the forward primer used in the RT-PCR amplification step is selected from SEQ ID NO: 18.
